(19) Europäisches Patentamt
European Patent Office
Office européen des brevets

(11) **EP 2 816 343 B1**

(12) **EUROPEAN PATENT SPECIFICATION**

(45) Date of publication and mention of the grant of the patent:
**23.11.2016 Bulletin 2016/47**

(21) Application number: **13749792.1**

(22) Date of filing: **14.02.2013**

(51) Int Cl.:
*G01N 21/15* (2006.01)  *G01N 21/25* (2006.01)
*G01N 21/85* (2006.01)  *G01N 33/28* (2006.01)
*G01N 21/94* (2006.01)

(86) International application number:
**PCT/JP2013/053495**

(87) International publication number:
**WO 2013/122129 (22.08.2013 Gazette 2013/34)**

(54) **LUBRICANT DETERIORATION SENSOR AND MACHINE PROVIDED WITH SAME**

SCHMIERMITTELVERSCHLECHTERUNGSSENSOR UND MIT DIESEM VERSEHENE MASCHINE

CAPTEUR DE DÉTÉRIORATION DE LUBRIFIANT ET MACHINE ÉQUIPÉE AVEC CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.02.2012  JP 2012032739**

(43) Date of publication of application:
**24.12.2014  Bulletin 2014/52**

(73) Proprietor: **Nabtesco Corporation
Tokyo 102-0093 (JP)**

(72) Inventors:
• **NAKAMURA, Koji
Tsu-shi, Mie 514-8533 (JP)**

• **SHIMADA, Hideshi
Tsu-shi, Mie 514-8533 (JP)**

(74) Representative: **Grünecker Patent- und
Rechtsanwälte
PartG mbB
Leopoldstraße 4
80802 München (DE)**

(56) References cited:
**EP-A1- 0 635 714      JP-A- H09 138 196
JP-A- H09 138 196     JP-A- H11 235 097
JP-A- 2005 156 297    JP-A- 2009 069 097
US-A1- 2008 024 761   US-A1- 2009 216 464**

**Description**

Technical Field

The present invention relates to a lubricant deterioration sensor for detecting the deterioration of lubricant of a machine.

Background Art

Conventionally, as a lubricant deterioration sensor for detecting the deterioration of lubricant of a machine, an oil deterioration degree sensor has been known. In this sensor, an oil entering gap portion for entering lubricant therein is formed on an optical path from an infrared LED (Light Emitting Diode) to a photo diode. Then, an amount of light absorption by the lubricant within the oil entering gap portion with respect to light emitted from the infrared LED is measured according to a light reception amount of the photo diode, to thereby determine a deterioration degree of the lubricant related to the light absorption amount thus measured (see patent literatures 1 and 2, for example).

However, the oil deterioration degree sensor described in each of the patent literatures 1 and 2 has a problem that, although a density of insoluble content within the lubricant can be measured as the deterioration degree of the lubricant, kinds of contaminant within the lubricant cannot be specified.

As a technique of specifying the kinds of contaminant within lubricant, a technique has been known in which an LED irradiates light toward a membrane filter having been used to filter the lubricant. Then, a light reception element converts reflection light from the contaminant on the membrane filter into digital values of RGB, to thereby specify the kinds of contaminant within the lubricant based on the RGB digital values thus converted (see non-patent literatures 1 and 2, for example). Patent Literature 3 concerns a method and apparatus for monitoring oil deterioration in real time. The apparatus includes a light source means for radiating light into an oil medium, a color sensing means for measuring light intensities of the light that has passed through a certain thickness of the oil.

Patent Literature 4 concerns an oil monitoring apparatus. In one embodiment, an oil monitoring apparatus includes a probe and an analyzing module in-line connected to the probe. The probe includes: a first sensor for measuring respective optical intensities of a light passing through the oil at respective red, green and blue wavelength ranges; a second sensor for measuring a water content; and a third sensor for measuring a temperature of the oil.

Citation List

Patent Literature

   PATENT LITERATURE 1: JP-A-7-146233
   PATENT LITERATURE 2: JP-A-10-104160
   PATENT LITERATURE 3: US 2008/024761 A1
   PATENT LITERATURE 4: US 2009/216464 A1
   NON-PATENT LITERATURE 1: Tomohiko Yamaguchi and four others, "METHOD OF DISCRIMINATING HUE OF CONTAMINANT WITHIN LUBRICANT", Fukui University,
   Faculty of Engineering, Research Report, March 2003, Volume 51, No. 1, pp. 81 - 88
   NON-PATENT LITERATURE 2: Tomomi Honda, "DETERIORATION DIAGNOSIS OF LUBRICANT •INSPECTION TECHNOLOGY", Journal of Japan Society for Precision Engineering, 2009, Volume 75, No. 3, pp. 359 - 362

Summary of Invention

Technical Problem

Since the technique described in each of the non-patent literatures 1 and 2 requires to drain lubricant from a machine and filter the lubricant by a membrane filter, there arises a problem of lacking immediacy.

Patent literatures 3 and 4 disclose a lubricant deterioration sensor as defined in the preamble of claim 1.

An object of this invention is to provide a lubricant deterioration sensor which enables to immediately specify the kinds and amounts of contaminant within lubricant of a machine, wherein degradation of the detection accuracy is suppressed.

Solution to Problem

According to this invention, there is provided with a lubricant deterioration sensor for detecting deterioration of lubricant of a machine body in a state where the lubricant deterioration sensor is mounted in the machine body, the lubricant deterioration sensor comprising:

a light emitting element configured to emit light;
a color light reception element configured to detect color of received light;
a gap forming member forming an oil gap into which the lubricant enters;
a supporting member supporting the light emitting element, the color light reception element and the gap forming member; and
a fixing member configured to be fixed to the machine body,

wherein
the gap forming member is configured to transmit the light emitted from the light emitting element,
the oil gap is disposed on an optical path from the light emitting element to the color light reception element,
the fixing member includes a contact portion that contacts with the machine body on an outer side of the contact portion, and
the supporting member is disposed on an inner side of the contact portion so as to be separated from the contact portion by a gap so that at least a part of the optical path is disposed on the inner side of the contact portion.

The contact portion may be configured to be inserted into a hole of the machine body.

The hole may be a screw hole, and the contact portion may be a screw portion which is configured to be inserted into the screw hole of the machine body and fixed thereto.

The fixing member may rotatably support the supporting member so that the direction of the opening of the oil gap changes when the supporting member rotates.

The supporting member may include a rotational driving force receiving portion, which receives a rotational driving force with respect to the fixing member from outside by a contact force, at a position where the rotational driving force receiving portion does not contact with the lubricant in a state where the fixing member is fixed to the machine body.

The lubricant deterioration sensor may further include a rotation preventing member contacting with both the supporting member and the fixing member so as to prevent a rotation of the supporting member with respect to the fixing member, wherein
the rotation preventing member includes a contact driving force receiving portion, which receives a driving force for contacting with both the supporting member and the fixing member from outside by a contact force, at a position where the rotational driving force receiving portion does not contact with the lubricant in a state where the fixing member is fixed to the machine body.

According to this invention, a machine is provided which includes the lubricant deterioration sensor and the machine body.

The machine may further include a cleaning member configured to clean the lubricant deterioration sensor. The machine body may include a sensor side portion at which the lubricant deterioration sensor is mounted and a cleaning member side portion which is movable with respect to the sensor side portion and at which the cleaning member is mounted. The cleaning member may be disposed at a position contacting with the gap forming surfaces of the gap forming member forming the oil gap in a case where the sensor side portion and the cleaning member side portion move relatively.

The cleaning member may be provided with a plurality of insertion portions configured to be inserted in the oil gap and having elasticity. The width of each of the insertion portions in the distance direction of the oil gap is smaller than the distance of the oil gap, and the entire width of the plurality of insertion portions in the distance direction of the oil gap is larger than the distance of the oil gap.

The machine may be a reducer for an industrial robot, and the machine body may be a main body of the reducer.

The machine may be the industrial robot. The machine body may include an arm and a reducer used at the articular portion of the arm, and the lubricant may be lubricant for the reducer.

The machine may be the industrial robot. The machine body may include an arm and a reducer used at the articular portion of the arm, and the lubricant may be lubricant for the reducer. The arm may be the sensor side portion. The reducer may be the cleaning member side portion. The cleaning member may be disposed at a position contacting with the gap forming surfaces when the reducer rotates.

Advantageous Effects of Invention

In the lubricant deterioration sensor according to this invention, the color light reception element detects colors with respect to the light having wavelengths not absorbed by the contaminant such as ion powder within the lubricant at the oil gap, among the light emitted by the light emitting element. Thus, colors of the contaminant within the lubricant of the machine body can be detected immediately. That is, the lubricant deterioration sensor according to this invention enables to immediately specify the kinds and amounts of the contaminant within the lubricant of the machine body based on the colors detected by the color light reception element. Further, in the lubricant deterioration sensor according to this invention, even if the contact portion is made in contact with the machine body and is deformed, the deformation of the contact potion is hardly transmitted to the supporting member which is disposed in separation from the contact portion. Thus, the change of the optical path due to the deformation of the supporting member hardly occurs. Accordingly, in the case where the contact portion is made in contact with the machine body, the detection accuracy of the deterioration of the lubricant of the machine body can be suppressed from degrading.

In the lubricant deterioration sensor according to this invention, the contact portion likely deforms toward the inside thereof when the contact portion is inserted into the hole of the machine body and made in contact with the machine body. Thus, in the case where the contact portion is made in contact with the machine body, the detection accuracy of the deterioration of the lubricant of the machine body can be efficiently suppressed from degrading.

In the lubricant deterioration sensor according to this invention, the screw portion likely deforms toward the inside thereof when the screw portion is threaded into the screw hole of the machine body and made in contact with the machine body. Thus, in the case where the screw portion is fixed to the machine body, the detection accuracy of the deterioration of the lubricant of the machine body can be efficiently suppressed from degrading.

In the lubricant deterioration sensor according to this invention, the direction of the opening of the oil gap at the time of fixing the fixing member to the machine body can be adjusted so that the detection accuracy of the deterioration of the lubricant of the machine body becomes high in a case where the fixing member is fixed to the machine body.

In the lubricant deterioration sensor according to this invention, the direction of the opening of the oil gap in the case of fixing the fixing member to the machine body can be adjusted so that the detection accuracy of the deterioration of the lubricant of the machine body becomes high after fixing the fixing member to the machine body.

In the lubricant deterioration sensor according to this invention, the direction of the opening of the oil gap in the case of fixing the fixing member to the machine body can be fixed so that the detection accuracy of the deterioration of the lubricant of the machine body becomes high after fixing the fixing member to the machine body.

The machine according to this invention can immediately specify the kinds and amounts of the contaminant within the lubricant of the machine body by using the lubricant deterioration sensor. Further, the machine according to this invention can suppress the degradation of the detection accuracy of deterioration of the lubricant of the machine body by the lubricant deterioration sensor in a case where the contact portion of the lubricant deterioration sensor contacts to the machine body.

In the machine according to this invention, when the sensor side portion and the cleaning member side portion move relatively to each other, the cleaning member rubs off dirt such as sludge adhered to the gap forming surfaces of the gap forming member. Thus, the degradation of performance of the lubricant deterioration sensor can be suppressed. As a result, the machine according to this invention can suppress the degradation of the performance of the lubricant deterioration sensor which enables to immediately specify the kinds and amounts of the contaminant within the lubricant.

In the machine according to this invention, as compared with a case where the cleaning member is configured only by a single insertion portion which width in the distance direction of the oil gap is larger than the distance of the oil gap, a pressure applied to the lubricant deterioration sensor by the cleaning member at the time of inserting the cleaning member in the oil gap can be made small. As a result, the degradation of performance of the lubricant deterioration sensor due to the pressure applied to the lubricant deterioration sensor from the cleaning member can be suppressed. Further, in the machine according to this invention, dirt such as sludge adhered to the gap forming surfaces of the gap forming member can be effectively rubbed off by the plurality of insertion portions of the cleaning member.

The reducer for the industrial robot according to this invention can suppress the degradation of the performance of the lubricant deterioration sensor which enables to immediately specify the kinds and amounts of the contaminant within the lubricant. Thus, the reducer for the industrial robot according to this invention can maintain for a long time the accuracy of the immediate prediction as to a failure.

The industrial robot according to this invention can suppress the degradation of the performance of the lubricant deterioration sensor which enables to immediately specify the kinds and amounts of the contaminant within the lubricant. Thus, the industrial robot according to this invention can maintain for a long time the accuracy of the immediate prediction as to a failure.

In the industrial robot according to this invention, the cleaning member can rub off dirt such as sludge adhered to the gap forming surfaces of the lubricant deterioration sensor each time the arm is driven by the reducer.

The lubricant deterioration sensor according to this invention enables to immediately specify the kinds and amounts of the contaminant within the lubricant of the machine.

Brief Description of Drawings

[Fig. 1] Fig. 1 is a side view of an industrial robot according to the first embodiment of the present invention.

[Fig. 2] Fig. 2 is a sectional view of the articular portion of the industrial robot shown in Fig. 1.

[Fig. 3] Fig. 3 is a front view of a lubricant deterioration sensor shown in Fig. 2.

[Fig. 4] Fig. 4 is a front sectional view of the lubricant deterioration sensor shown in Fig. 3 in an attached state to an arm.

[Fig. 5] Fig. 5(a) is a plan view of the lubricant deterioration sensor shown in Fig. 3. Fig. 5(b) is a bottom view of the lubricant deterioration sensor shown in Fig. 3.

[Fig. 6] Fig. 6(a) is a front view of a housing shown in Fig. 3. Fig. 6(b) is a front sectional view of the housing shown in Fig. 3.

[Fig. 7] Fig. 7(a) is a side view of the housing shown in Fig. 3. Fig. 7(b) is a side sectional view of the housing shown in Fig. 3.

[Fig. 8] Fig. 8(a) is a plan view of the housing shown in Fig. 3. Fig. 8(b) is a bottom view of the housing shown in Fig. 3.

[Fig. 9] Fig. 9(a) is a front view of a holder shown in Fig. 3. Fig. 9(b) is a front sectional view of the holder shown in Fig. 3.

[Fig. 10] Fig. 10(a) is a side view of the holder shown in Fig. 3. Fig. 10(b) is a side sectional view of the holder shown in Fig. 3.

[Fig. 11] Fig. 11 (a) is a plan view of the holder shown in Fig. 3. Fig. 11 (b) is a bottom view of the holder shown in Fig. 3.

[Fig. 12] Fig. 12 is a diagram showing an optical path from a white LED to an RGB sensor shown in Fig. 4.

[Fig. 13] Fig. 13(a) is a front view of a holder cap shown in Fig. 3. Fig. 13(b) is a front sectional view of the holder cap shown in Fig. 3.

[Fig. 14] Fig. 14(a) is a plan view of the holder cap shown in Fig. 3. Fig. 14(b) is a bottom view of the holder cap shown in Fig. 3.

[Fig. 15] Fig. 15 is a diagram showing an example of the relation between the direction of the opening of an oil gap shown in Fig. 3 with respect to the flow of lubricant and a color difference $\Delta E$ of colors detected by the RGB sensor with respect to black.

[Fig. 16] Fig. 16(a) is a diagram showing a state that the direction of the opening of the oil gap shown in Fig. 3 with respect to the flow of the lubricant is 0 degree. Fig. 16(b) is a diagram showing a state that the direction of the opening of the oil gap shown in Fig. 3 with respect to the flow of the lubricant is 45 degrees. Fig. 16(c) is a diagram showing a state that the direction of the opening of the oil gap shown in Fig. 3 with respect to the flow of the lubricant is 90 degrees.

[Fig. 17] Fig. 17(a) is a table showing the experimentation result of the chronological change of the color difference $\Delta E$ in a case where the lubricant shown in Fig. 2 contains molybdenum as additive. Fig. 17(b) is a graph of the experimentation result shown in Fig. 17(a).

[Fig. 18] Fig. 18(a) is a table showing the experimentation result of the chronological change of the color difference $\Delta E$ in a case where the lubricant shown in Fig. 2 does not contain molybdenum as additive. Fig. 18(b) is a graph of the experimentation result shown in Fig. 18(a).

[Fig. 19] Fig. 19 is a sectional view of the articular portion of an industrial robot according to the second embodiment of this invention.

[Fig. 20] Fig. 20 is a plan view of the component of the supporting body shown in Fig. 19 to which cleaning members are fixed.

[Fig. 21] Fig. 21(a) is a plan view of an example of the cleaning member shown in Fig. 20. Fig. 21(b) is a plan view of another example of the cleaning member different from that shown in Fig. 21 (a).

[Fig. 22] Fig. 22(a) is a plan view of the cleaning member in a case where a part of the cleaning member shown in Fig. 21(a) is inserted in an oil gap. Fig. 22(b) is a plan view of the cleaning member in a case where a part of the cleaning member shown in Fig. 21(b) is inserted in the oil gap.

[Fig. 23] Fig. 23 is a perspective view of the cleaning member in a case where an insertion portion shown in Fig. 21 is a brush.

[Fig. 24] Fig. 24 is a sectional diagram showing the articular portion of the industrial robot according to the second embodiment of this invention, which shows another example of the articular portion different from that shown in Fig. 19.

Description of Embodiments

Hereinafter, embodiments of this invention will be explained with reference to drawings.

First, the configuration of an industrial robot as a machine according to the first embodiment will be explained.

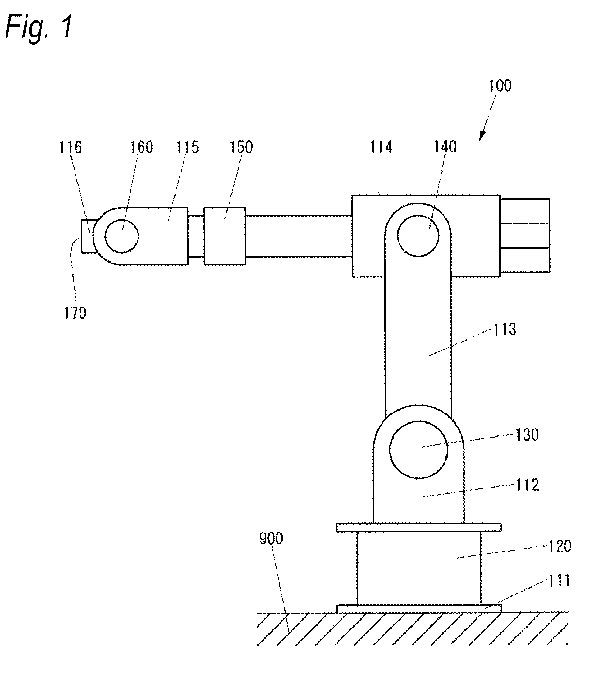

Fig. 1 is a side view of the industrial robot 100 according to this embodiment.

As shown in Fig. 1, the industrial robot 100 includes an attachment portion 111 to be attached to installation placing portion 900 such as a floor or a ceiling, arms 112 to 116, an articular portion 120 for connecting between the attachment portion 111 and the arm 112, an articular portion 130 for connecting between the arm 112 and the arm 113, an articular portion 140 for connecting between the arm 113 and the arm 114, an articular portion 150 for connecting between the arm 114 and the arm 115, an articular portion 160 for connecting between the arm 115 and the arm 116, and an articular portion 170 for connecting between the arm 116 and a not-shown hand.

Of the industrial robot 100, portions except for lubricant such as lubricant 131 a described later, the lubricant deterioration sensors such as lubricant deterioration sensors 137a, 137b, 139a, 139b described later constitute a machine body according to this invention in a case where the industrial robot 100 is the machine according to this invention.

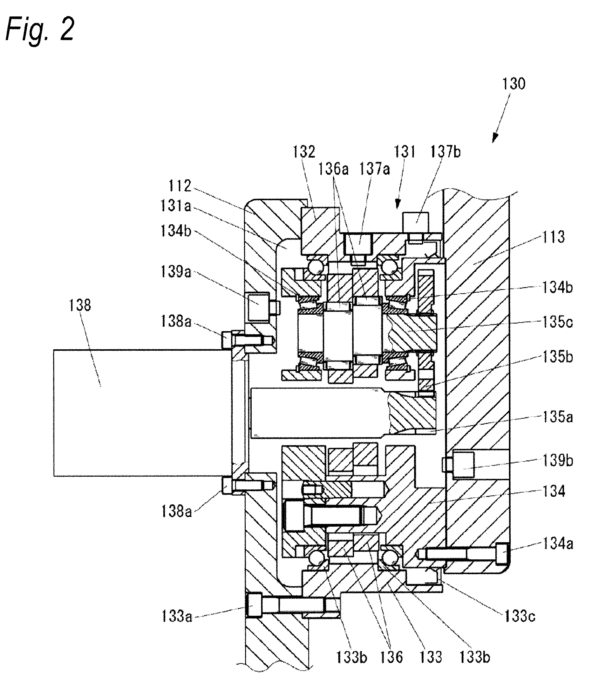

Fig. 2 is a sectional view of the articular portion 130. Although the explanation is made hereinafter as to the articular portion 130, the configuration of each of the articular portions 120, 140 to 170 is substantially same.

As shown in Fig. 2, the articular portion 130 includes a reducer 131 for connecting between the arm 112 and the arm 113, a motor 138 fixed to the arm 112 by means of bolts 138a, and the lubricant deterioration sensors 139a, 139b each for detecting the deterioration of the lubricant 131a for reducing friction generated at the movable portions of the reducer 131.

The reducer 131 includes a reducer body 132 and the lubricant deterioration sensors 137a and 137b each for detecting the deterioration of the lubricant 131a of the reducer body 132. The reducer body 132 constitutes a machine body according to this invention in a case where the reducer 131 is the machine according to this invention.

The reducer body 132 includes a case 133 fixed to the arm 112 by means of bolts 133a; a supporting body 134 fixed to the arm 113 by means of bolts 134a; a gear 135a fixed to the output shaft of the motor 138; three gears 135b which are disposed around the center axis of the reducer 131 with a constant interval therebetween and mesh with the gear 135a; three crank shafts 135c which are disposed around the center axis of the reducer 131 with a constant interval therebetween and fixed to the gears 135b, respectively; and two external gears 136 which mesh with internal gears provided at the case 133.

The supporting body 134 is rotatably supported by the case 133 via bearings 133b. A seal member 133c for preventing leakage of the lubricant 131 a is provided between the case 133 and the supporting body 134.

Each of the crank shafts 135c is rotatably supported by the supporting body 134 via bearings 134b and further rotatably supported by the external gears 136 via bearings 136a.

Each of the lubricant deterioration sensor 137a and the lubricant deterioration sensor 137b is fixed to the case 133. The lubricant deterioration sensor 139a is fixed to the arm 112. The lubricant deterioration sensor 139b is fixed to the arm 113.

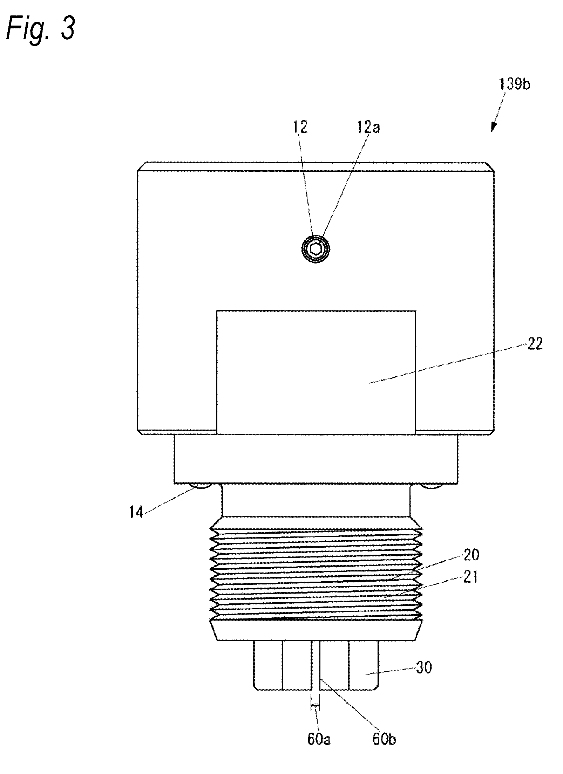

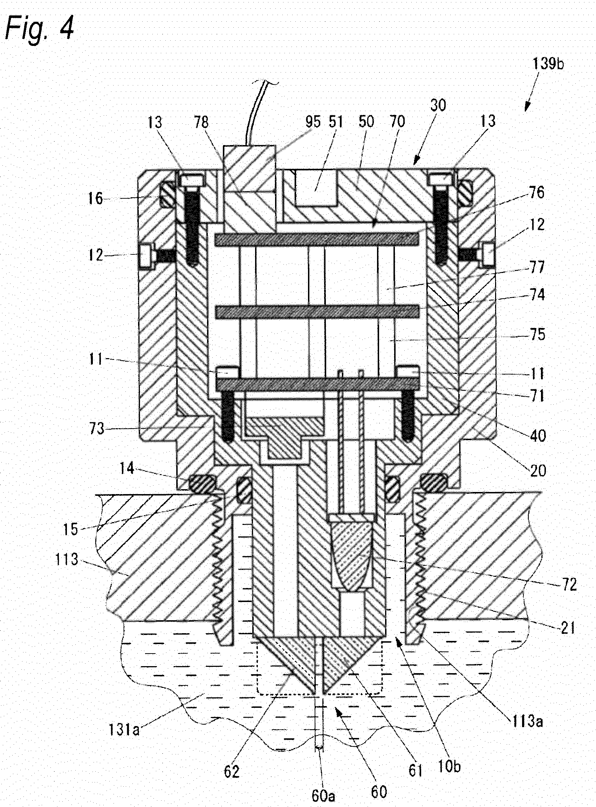

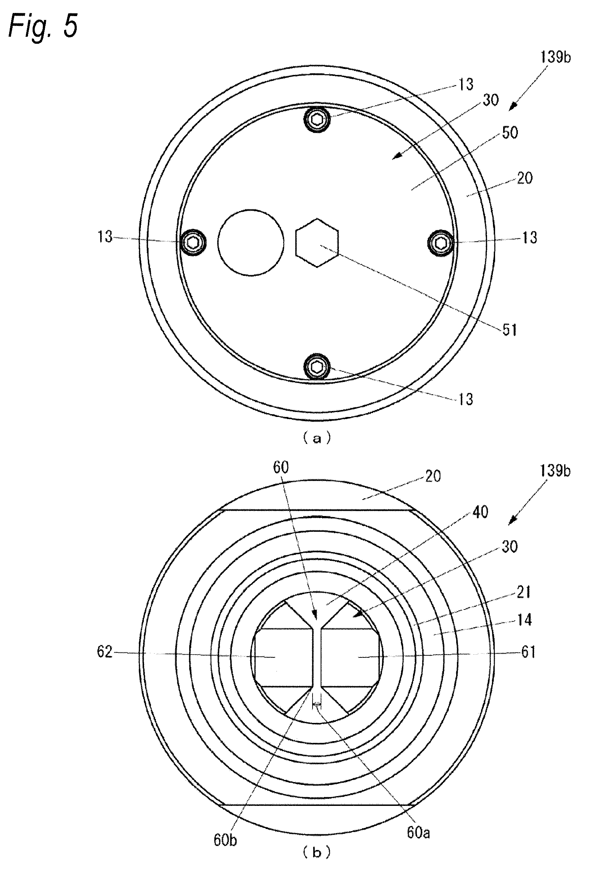

Fig. 3 is a front view of the lubricant deterioration sensor 139b. Fig. 4 is a front sectional view of the lubricant deterioration sensor 139b in an attached state to the arm 113. Fig. 5(a) is a plan view of the lubricant deterioration sensor 139b. Fig. 5(b) is a bottom view of the lubricant deterioration sensor 139b. Although the explanation is made hereinafter as to the lubricant deterioration sensor 139b, the configuration of each of the lubricant deterioration sensors such as the lubricant deterioration sensors 137a, 137b, 139a other than the lubricant deterioration sensor 139b is substantially same.

As shown in Figs. 3 to 5(b), the lubricant deterioration sensor 139b includes a housing 20 made of aluminum alloy for supporting the respective components of the lubricant deterioration sensor 139b; a supporting member 30 for supporting a white LED 72, an RGB sensor 73 and a gap forming member 60 described later; the gap forming member 60 held by the supporting member 30; and an electronic component group 70 having the white LED 72 and the RGB sensor 73.

The supporting member 30 is fixed to the housing 20 by means of bolts 12 with hexagon holes. The supporting member 30 includes a holder 40 made of aluminum alloy, and a holder cap 50 made of aluminum alloy which is fixed to the holder 40 by means of bolts 13 with hexagon holes.

The gap forming member 60 is constituted by two right-angle prisms 61, 62 made of glass. An oil gap 60a as a gap for entering the lubricant 131 a therein is formed between the two right-angle prisms 61, 62.

The electronic component group 70 includes a circuit board 71 fixed to the supporting member 30 by means of screws 11; the white LED 72 mounted on the circuit board 71; the RGB sensor 73 mounted on the circuit board 71; a circuit board 74 disposed at one surface side of the circuit board 71 opposing to the white LED 72 and RGB sensor 73 side thereof; a plurality of pillars 75 for fixing the circuit board 71 and the circuit board 74; a circuit board 76 disposed on the opposite surface side of the circuit board 71 with respect to the circuit board 74; a plurality of pillars 77 for fixing the circuit board 74 and the circuit board 76; and a connector 78 mounted on one surface side of the circuit board 76 opposing to the circuit board 74 side thereof. A plurality of electronic components are mounted on each of the circuit board 71, the circuit board 74 and the circuit board 76. The circuit board 71, the circuit board 74 and the circuit board 76 are mutually connected electrically.

The lubricant deterioration sensor 139b includes an O ring 14 for preventing the leakage of the lubricant 131 a from a gap between the housing 20 and the arm 113, an O ring 15 for preventing the leakage of the lubricant 131 a from a gap between the housing 20 and the holder 40, and an O ring 16 disposed between the housing 20 and the holder cap 50.

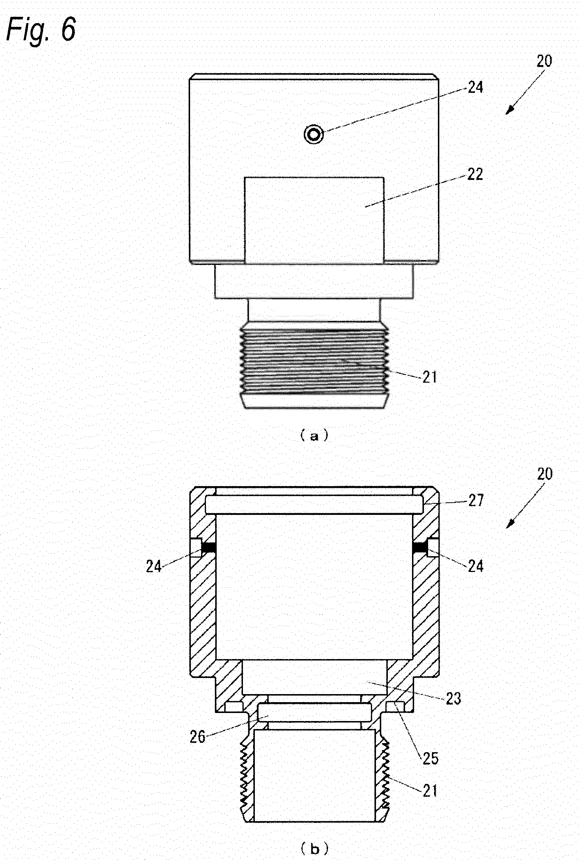

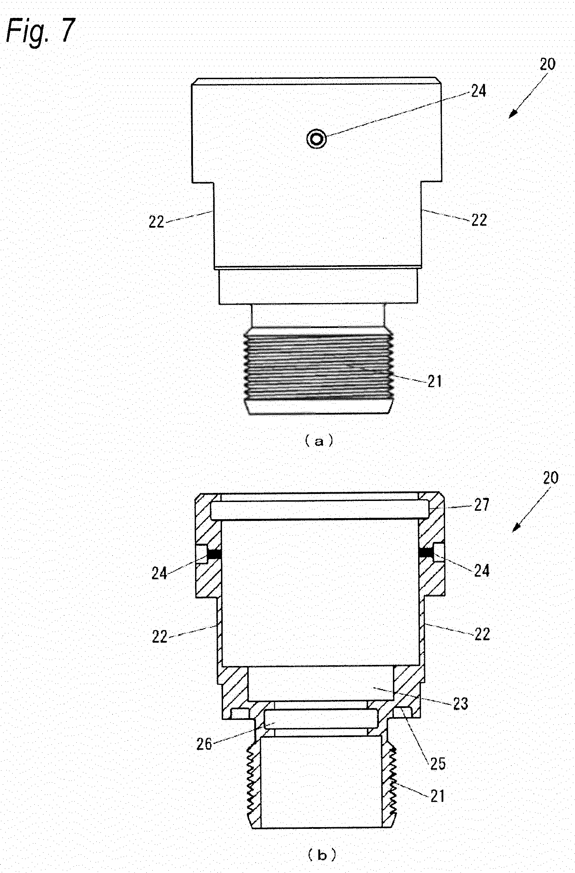

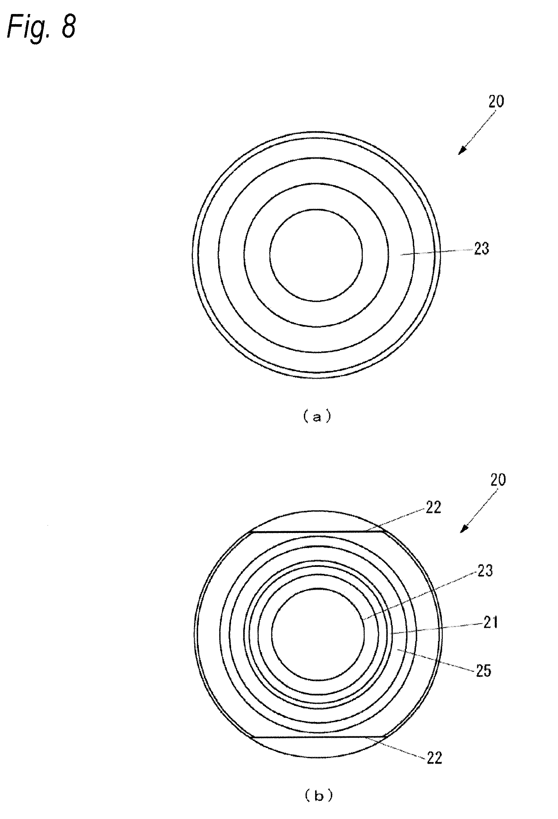

Fig. 6(a) is a front view of the housing 20. Fig. 6(b) is a front sectional view of the housing 20. Fig. 7(a) is a side view of the housing 20. Fig. 7(b) is a side sectional view of the housing 20. Fig. 8(a) is a plan view of the housing 20. Fig. 8(b) is a bottom view of the housing 20.

As shown in Figs. 3 to 8(b), the housing 20 includes a screw portion 21 to be fixed to the screw hole 113a of the arm 113, a tool contact portion 22 which is grasped by a tool such as a wrench at the time of rotating the screw portion 21 with respect to the screw hole 113a of the arm 113, and a holder housing portion 23 in which the holder 40 is housed. Further, the housing 20 is provided with screw holes 24 into which the bolts 12 with hexagon holes are respectively screwed, a groove 25 into which the O ring 14 is fit, a groove 26 into which the O ring 15 is fit, and a groove 27 into which the O ring 16 is fit.

The housing 20 is configured to be fixed to the arm 113 of the industrial robot 100, that is, the machine body and hence constitutes a fixing member of this invention. Further, the screw portion 21 is configured to be inserted into the screw hole 113a of the arm 113 and fixed to the screw hole 113a on the outer side thereof, and constitutes a contact portion according to this invention.

The screw hole 113a of the arm 113 may be used for supplying the lubricant 131a to the reducer 131 and for disposing the lubricant 131a from the reducer 131 in a state that the lubricant deterioration sensor 139b is removed.

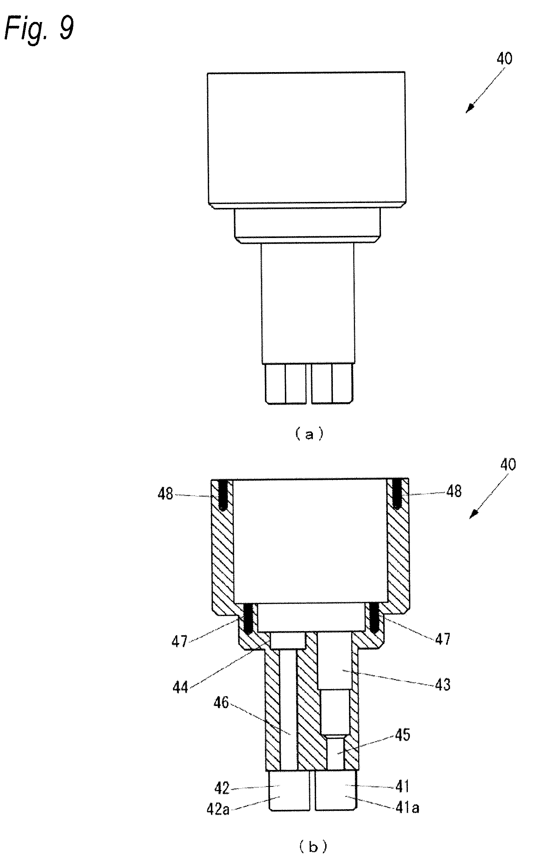

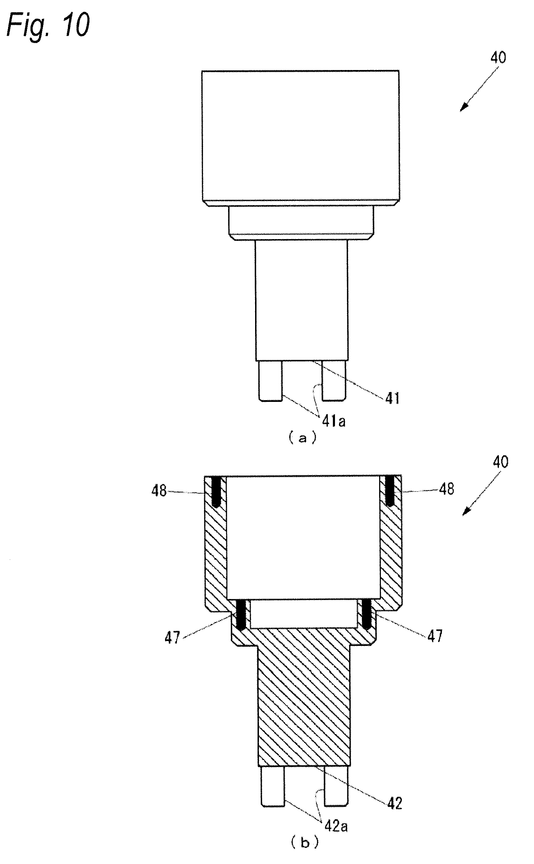

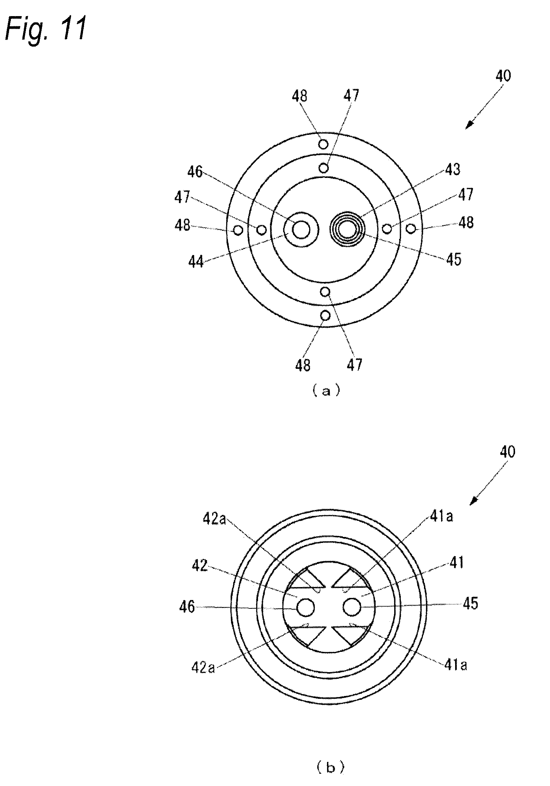

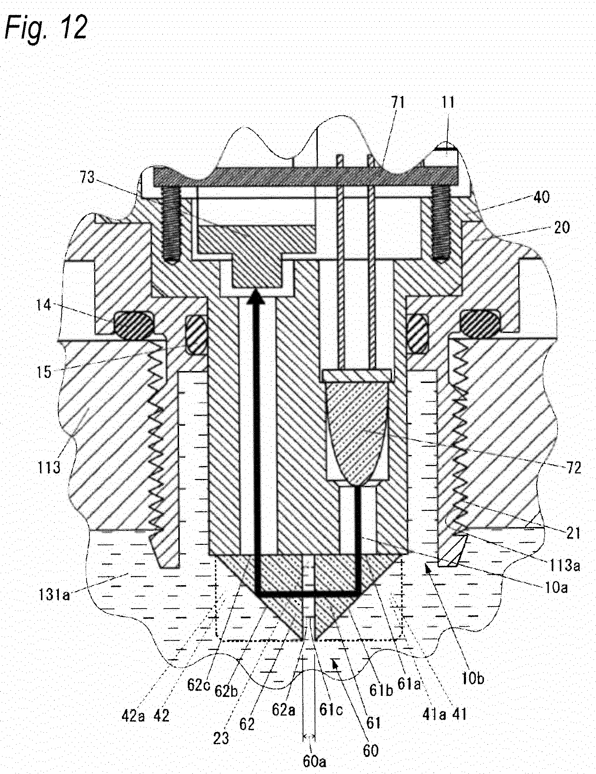

Fig. 9(a) is a front view of the holder 40. Fig. 9(b) is a front sectional view of the holder 40. Fig. 10(a) is a side view of the holder 40. Fig. 10(b) is a side sectional view of the holder 40. Fig. 11 (a) is a plan view of the holder 40. Fig. 11 (b) is a bottom view of the holder 40. Fig. 12 is a diagram showing an optical path 10a from the white LED 72 to the RGB sensor 73.

As shown in Figs.3 to 5(b) and Figs. 9(a) to 12, the holder 40 includes a prism housing portion 41 for housing the right-angle prism 61, a prism housing portion 42 for housing the right-angle prism 62, an LED housing portion 43 for housing the white LED 72, and an RGB sensor housing portion 44 for housing the RGB sensor 73. Further, the holder 40 is provided with a hole 45 penetrating the prism housing portion 41 and the LED housing portion 43, a hole 46 penetrating the prism housing portion 42 and the RGB sensor housing portion 44, screw holes 47 in which the screws 11 are threaded, and screw holes 48 in which the bolts 13 with hexagon holes are threaded.

The prism housing portion 41 includes two walls 41a sandwiching the right-angle prism 61 therebetween. The right-angle prism 61 is fixed to the walls 41 a by means of adhesive. The prism housing portion 42 includes two walls 42a sandwiching the right-angle prism 62 therebetween. The right-angle prism 62 is fixed to the walls 42a by means of adhesive.

The holder 40 surrounds at least a part of the optical path 10a from the white LED 72 to the RGB sensor 73 by means of the LED housing portion 43, the hole 45, the prism housing portion 41, the prism housing portion 42, the hole 46 and the RGB sensor housing portion 44.

The surface of the holder 40 is treated to a treatment for preventing light reflection such as a black alumite treatment for matting.

The holder 40 supports the white LED 72 and the RGB sensor 73 via the circuit board 71. Further, the holder 40 directly supports the gap forming member 60.

As shown in Fig. 12, the oil gap 60a of the gap forming member 60 is disposed on the optical path 10a from the white LED 72 to the RGB sensor 73.

The right-angle prisms 61, 62 transmit light emitted from the white LED 72. The right-angle prism 61 is provided with a light incident surface 61 a on which light emitted from the white LED 72 is made incident, a light reflection surface 61 b which reflects the light entered from the light incident surface 61 a in a manner of bending the propagation direction of the light by 90 degrees, and a light emission surface 61 c which emits the light reflected by the light reflection surface 61 b. The right-angle prism 62 is provided with a light incident surface 62a on which light emitted from the light emission surface 61c of the right-angle prism 61 is made incident, a light reflection surface 62b which reflects the light entered from the light incident surface 62a in a manner of bending the propagation direction of the light by 90 degrees, and a light emission surface 62c which emits the light reflected by the light reflection surface 62b.

Each of the light incident surface 61a, the light reflection surface 61b and the light emission surface 61 c of the right-angle prism 61 and the light incident surface 62a, the light reflection surface 62b and the light emission surface 62c of the right-angle prism 62 is optically polished. Further, each of the light reflection surface 61 b of the right-angle prism 61 and the light reflection surface 62b of the right-angle prism 62 is provided with an aluminum deposition film. Furthermore, an $SiO_2$ film is formed on the aluminum deposition film in order to protect the aluminum deposition film which is low in a degree of hardness and an adhesive force.

The optical path 10a is bent by 90 degrees by the light reflection surface 61b of the right-angle prism 61 and also bent by 90 degrees by the light reflection surface 62b of the right-angle prism 62. That is, the optical path 10a is bent by 180 degrees by the gap forming member 60.

When a distance between the light emission surface 61 c of the right-angle prism 61 and the light incident surface 62a of the right-angle prism 62, that is, the length of the oil gap 60a is too short, the contaminant within the lubricant 131a unlikely flows suitably through the oil gap 60a. Thus, the detection accuracy of the colors of the contaminant within the lubricant 131 a degrades. On the other hand, when the length of the oil gap 60a is too long, the light emitted from the white LED 72 is excessively absorbed by the contaminant within the lubricant 131 a within the oil gap 60a and hence unlikely reaches the RGB sensor 73. Thus, the detection accuracy of the colors of the contaminant within the lubricant 131 a also degrades. Accordingly, preferably, the length of the oil gap 60a is set suitably so that the detection accuracy of the colors of the contaminant within the lubricant 131a becomes high. The length of the oil gap 60a is 1 mm, for example.

The white LED 72 is an electronic component which emits white light and constitutes a light emitting element according to this invention. As the white LED 72, NSPW500GS-K1 manufactured by Nichia Corporation, for example, may be employed.

The RGB sensor 73 is an electronic component which detects the colors of the received light and constitutes a color light reception element according to this invention. As the RGB sensor 73, S9032-02 manufactured by Hamamatsu Photonics K.K., for example, may be employed.

As shown in Fig. 4, the connector 78 is configured so that the connector 95 of the external device at the outside of the lubricant deterioration sensor 139b is connected thereto so as to be supplied with electric power via the connector 95 from the external device, and the detection result of the lubricant deterioration sensor 139b is outputted as an electric signal to the external device via the connector 95.

As shown in Fig. 12, the holder 40 is disposed on the inner side of the screw portion 21 so as to be separated from the screw portion 21 so that a part of the optical path 10a is disposed on the inner side of the screw portion 21 of the housing 20. In other words, a gap 10b is formed between the screw portion 21 of the housing 20 and the holder 40.

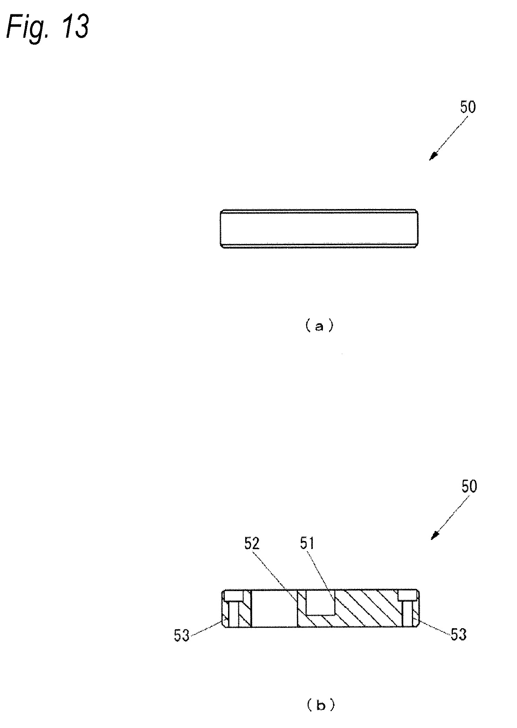

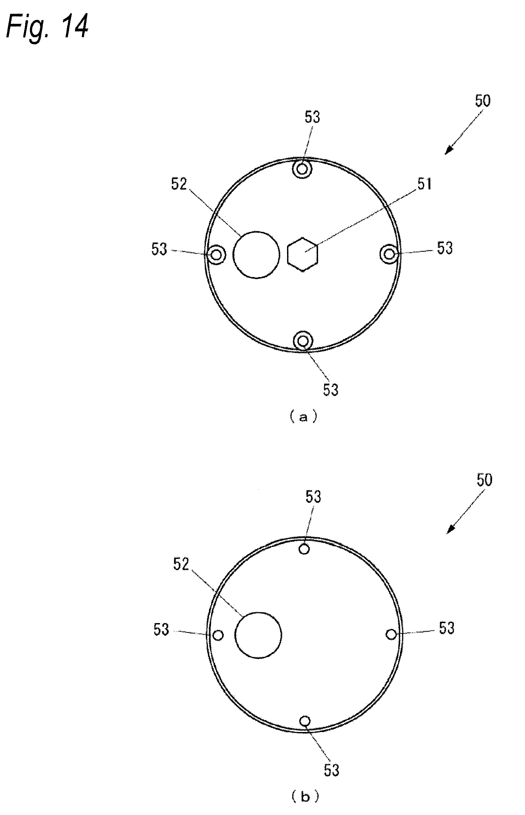

Fig. 13(a) is a front view of the holder cap 50. Fig. 13(b) is a front sectional view of the holder cap 50. Fig. 14(a) is a plan view of the holder cap 50. Fig. 14(b) is a bottom view of the holder cap 50.

As shown in Figs. 3 to 5(b) and Figs. 13(a) to 14(b), the holder cap 50 is provided with a tool contact portion 51 for contacting with a tool such as a hexagonal wrench at the time of rotating the supporting member 30 with respect to the housing 20. The tool contact portion 51 is a portion for receiving a rotational driving force of the supporting member 30 with respect to the housing 20 from the outside by a contact force, and constitutes a rotational driving force receiving portion according to this invention. The tool contact portion 51 is disposed at a position not contacting with the lubricant 131 a when the housing 20 is fixed to the arm 113. Further, the holder cap 50 is provided with a hole 52 in which the connector 78 is inserted and holes 53 in which the bolts 13 with hexagon holes are inserted.

The surface of the holder cap 50 is treated to the treatment for preventing light reflection such as the black alumite treatment for matting.

As shown in Figs. 3 and 4, the bolts 12 with hexagon holes are configured so as to prevent the rotation of the supporting member 30 with respect to the housing 20 by contacting with both the supporting member 30 and the housing 20, and constitute a rotation preventing member according to this invention. The bolt 12 with a hexagon hole has a tool contact portion 12a for contacting with a tool such as the hexagonal wrench. The tool contact portion 12a is a portion for receiving a driving force for contacting with both the supporting member 30 and the housing 20 from the outside by a contact force, and constitutes a contact driving force receiving portion according to this invention. The tool contact portion 12a is disposed at a position not contacting with the lubricant 131 a when the housing 20 is fixed to the arm 113.

Next, the assembling method of the lubricant deterioration sensor 139b will be explained. Although the explanation is made hereinafter as to the lubricant deterioration sensor 139b, the assembling method of each of the lubricant deterioration sensors such as the lubricant deterioration sensors 137a, 137b, 139a other than the lubricant deterioration sensor 139b is substantially same.

First, adhesive is pasted on the surface plane of the prism housing portion 41 of the holder 40 to be made in contact with the light incident surface 61 a of the right-angle prism 61, and also adhesive is pasted on the two surface planes of the right-angle prism 61 to be made in contact with the two walls 41a of the prism housing portion 41. Then, the right-angle prism 61 is fixed to the prism housing portion 41 by the adhesive. Further, adhesive is pasted on the surface plane of the prism housing portion 42 of the holder 40 to be made in contact with the light emission surface 62c of the right-angle prism 62, and also adhesive is pasted on the two surface planes of the right-angle prism 62 to be made in contact with the two walls 42a of the prism housing portion 42. Then, the right-angle prism 62 is fixed to the prism housing portion 42 by the adhesive. Furthermore, the white LED 72 is fixed to the LED housing portion 43 of the holder 40 by means of adhesive.

Then, the circuit board 71 mounting the RGB sensor 73 thereon is fixed to the holder 40 by means of the screws 11, and the white LED 72 is fixed to the circuit board 71 by means of soldering. Further, various kinds of electronic components such as the connector 78 are assembled, thereby supporting the electronic component group 70 by the holder 40.

Next, the holder cap 50 is fixed to the holder 40 by means of the bolts 13 with hexagon holes.

Lastly, the holder 40 is fixed, by means of the bolts 12 with hexagon holes, to the holder housing portion 23 of the housing 20 attached with the O ring 14, the O ring 15 and the O ring 16.

Next, the explanation will be made as to a method of mounting the lubricant deterioration sensor 139b to the arm 113. Although the explanation is made hereinafter as to the lubricant deterioration sensor 139b, the mounting method of each of the lubricant deterioration sensors such as the lubricant deterioration sensors 137a, 137b, 139a other than the lubricant deterioration sensor 139b is substantially same.

First, the tool contact portion 22 of the housing 20 is grasped by a tool and the screw portion 21 of the housing 20 is inserted into the screw hole 113a of the arm 113. That is, the lubricant deterioration sensor 139b is fixed to the arm 113 by threading the screw portion into the screw hole.

Then, the connector 95 of the external device at the outside of the lubricant deterioration sensor 139b is connected to the connector 78.

Next, the operation of the industrial robot 100 will be explained.

First, the operation of the articular portion 130 will be explained. Although the explanation is made hereinafter as to the articular portion 130, the operation of each of the articular portions 120, 140 to 170 is substantially same.

When the output shaft of the motor 138 of the articular portion 130 rotates, the rotation speed of the motor 138 is reduced by the reducer 131, whereby the arm 113 fixed to the supporting body 134 of the reducer 131 is moved by the rotation force of the motor with respect to the arm 112 fixed to the case 133 of the reducer 131.

Next, the operation of the lubricant deterioration sensor 139b will be explained. Although the explanation is made hereinafter as to the lubricant deterioration sensor 139b, the operation of each of the lubricant deterioration sensors such as the lubricant deterioration sensors 137a, 137b, 139a other than the lubricant deterioration sensor 139b is substantially same.

The lubricant deterioration sensor 139b emits white light from the white LED 72 in response to electric power supplied from the external device via the connector 78.

Then, the lubricant deterioration sensor 139b outputs light amounts of respective colors RGB of the light received by the RGB sensor 73 as an electric signal to the external device via the connector 78.

The lubricant deterioration sensor 139b may additionally mount another sensor other than the RGB sensor 73. For example, in the lubricant deterioration sensor 139b, when a temperature sensor for detecting the temperature of the lubricant 131a is contained in the electronic component group 70, the temperature detected by the temperature sensor can also be outputted as an electric signal to the external device via the connector 78.

Next, the explanation will be made as to a method of adjusting the direction of the opening 60b of the oil gap 60a of the lubricant deterioration sensor 139b. Although the explanation is made hereinafter as to the lubricant deterioration sensor 139b, the adjusting method of each of the lubricant deterioration sensors such as the lubricant deterioration sensors 137a, 137b, 139a other than the lubricant deterioration sensor 139b is substantially same.

The external device of the lubricant deterioration sensor 139b can specify the kinds and amounts of the contaminant within the lubricant 131 a of the reducer 131 based on the colors detected by the RGB sensor 73. That is, the lubricant deterioration sensor 139b can detect the deterioration degree of the lubricant 131 a by detecting the colors of the contaminant within the lubricant 131 a.

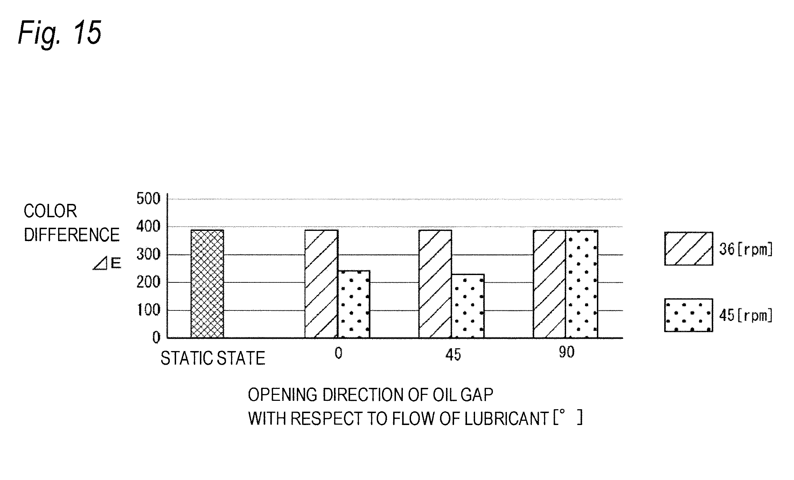

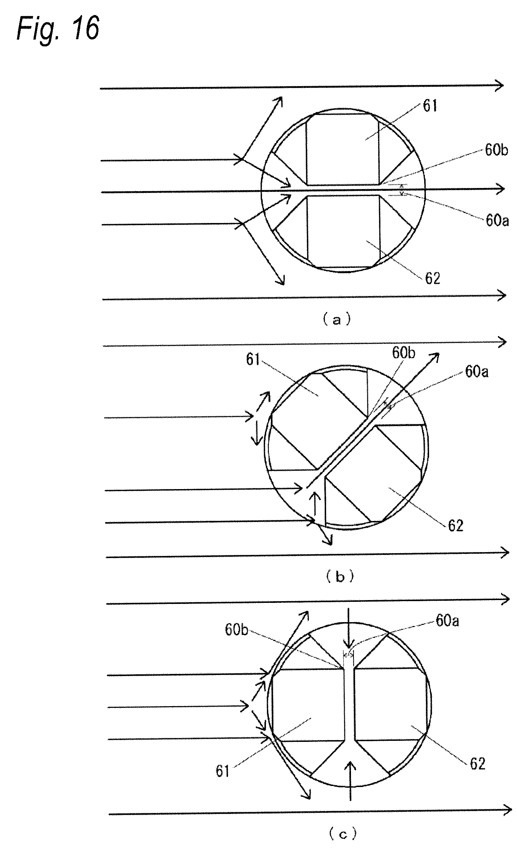

Fig. 15 is a diagram showing an example of the relation between the direction of the opening 60b of the oil gap 60a with respect to the flow of the lubricant 131a and a color difference ΔE of the colors detected by the RGB sensor 73 with respect to black. Fig. 16(a) is a diagram showing a state that the direction of the opening 60b of the oil gap 60a with respect to the flow of the lubricant 131 a is 0 degree. Fig. 16(b) is a diagram showing a state that the direction of the opening 60b of the oil gap 60a with respect to the flow of the lubricant 131a is 45 degrees. Fig. 16(c) is a diagram showing a state that the direction of the opening 60b of the oil gap 60a with respect to the flow of the lubricant 131a is 90 degrees.

The color difference ΔE of the colors detected by the RGB sensor 73 with respect to black can be calculated by an expression shown by the following Math. 1 by using the respective values of the colors RGB detected by the RGB sensor 73.

[Math. 1]

$$\Delta E = \sqrt{R^2 + G^2 + B^2}$$

In the experiment where the relation shown in Fig. 15 was derived, new oil with a low deterioration degree was used as the lubricant 131 a.

Further, in Fig. 15, a "static state" represents a time period where the flow of the lubricant 131a stops. When the flow of the lubricant 131a stops, the direction of the opening 60b of the oil gap 60a with respect to the flow of the lubricant 131 a does not influence on the ΔE of the colors detected by the RGB sensor 73 with respect to black. Thus, the ΔE of the colors detected by the RGB sensor 73 with respect to black in the "static state" becomes a determination criterion of the relation between the direction of the opening 60b of the oil gap 60a with respect to the flow of the lubricant 131 a and the color difference ΔE of the colors detected by the RGB sensor 73 with respect to black.

Further, in Fig. 15, each of 36 [rpm] and 45 [rpm] represents the rotation speed of the arm 113 with respect to the arm 112 as a revolution number per one minute. Since the lubricant deterioration sensor 139b is attached to the arm 113, this sensor moves within the lubricant 131a in accordance with the rotation of the arm 113 with respect to the arm 112. In other words, each of 36 [rpm] and 45 [rpm] indirectly represents the speed of the flow of the lubricant 131a with respect to the lubricant deterioration sensor 139b.

In Figs. 16(a) to 16(c), arrows other than those representing the oil gap 60a represent the flow of the lubricant 131 a.

The detection accuracy of the deterioration of the lubricant 131a can be determined by the color difference $\Delta E$ of the colors detected by the RGB sensor 73 with respect to black. In other words, in Fig. 15, the detection accuracy of the deterioration of the lubricant 131 a degrades in a case that the rotation speed of the arm 113 with respect to the arm 112 is 45 [rpm] and the direction of the opening 60b of the oil gap 60a with respect to the flow of the lubricant 131 a is 0 degree or 45 degrees. In this manner, the detection accuracy of the deterioration of the lubricant 131 a degrades depending on the direction of the opening 60b of the oil gap 60a with respect to the flow of the lubricant 131a.

The lubricant deterioration sensor 139b is configured to be adjustable in the direction of the opening 60b of the oil gap 60a.

First, each of the bolts 12 with hexagon holes is loosened by a tool inserted into the tool contact portion 12a so that the supporting member 30 becomes rotatable with respect to the housing 20.

Then, in a state that the rotation of the housing 20 with respect to the arm 113 is prevented by grasping the tool contact portion 22 of the housing 20 by the tool, the supporting member 30 is rotated with respect to the housing 20 by the tool inserted into the tool contact portion 51. The direction of the opening 60b of the oil gap 60a changes in accordance with the rotation of the supporting member 30 with respect to the housing 20.

Lastly, each of the bolts 12 with hexagon holes is fastened by the tool inserted into the tool contact portion 12a so that the rotation of the supporting member 30 becomes impossible with respect to the housing 20.

As explained above, the RGB sensor 73 detects colors with respect to the light having wavelengths not absorbed by the contaminant within the lubricant 131 a at the oil gap 60a, among the white light emitted by the white LED 72. Thus, each of the lubricant deterioration sensors such as the lubricant deterioration sensor 139b can immediately detect the colors of the contaminant within the lubricant 131 a of the reducer 131. That is, each of the lubricant deterioration sensors can immediately specify the kinds and amounts of the contaminant within the lubricant 131a of the reducer 131 based on the colors detected by the RGB sensor 73, by using the external device such as a computer. Each of the lubricant deterioration sensors may be configured that the electronic component group 70 contains an electronic component which specifies the kinds and amounts of the contaminant within the lubricant based on the colors detected by the RGB sensor 73.

Generally, in the industrial robot, accuracy of the track of the arm etc. largely depends on the performance of the reducer used at the articular portion. Thus, it is important to suitably exchange the reducer for the industrial robot for new one when the performance of the reducer degrades. However, in the case of exchanging the reducer for the industrial robot, it is required to stop the industrial robot provided with this reducer and a production line mounting the industrial robot. Thus, in order to grasp the exchange time of the reducer for the industrial robot, it is very important to suitably predict a failure of the reducer for the industrial robot. In this respect, as described above, each of the lubricant deterioration sensors of the industrial robot 100 enables to immediately specify the kinds and amounts of the contaminant within the lubricant 131a of the reducer 131 based on the colors detected by the RGB sensor 73, by using the external device such as a computer. Thus, the industrial robot 100 and each of the reducers of the industrial robot 100 enables to perform immediate prediction of a failure.

In each of the lubricant deterioration sensors such as the lubricant deterioration sensor 139b, the supporting member 30 is disposed on the inner side of the screw portion 21 so as to be separated from the screw portion 21 so that a part of the optical path 10a is disposed on the inner side of the screw portion 21. Thus, for example, in a case where a screw hole such as the screw hole 113a is formed to be small as compared with the screw portion 21, even if the screw portion 21 of the housing 20 is made in contact on the outer side thereof with the screw hole of the machine body such as the screw hole 113a of the arm 113 and is deformed toward the inner side, the deformation of the screw portion 21 is hardly transmitted to the supporting member 30 which is disposed in separation from the screw portion 21. Accordingly, the change of the optical path 10a due to the deformation of the supporting member 30 hardly occurs. As a result, in the case where each of the lubricant deterioration sensors such as the lubricant deterioration sensor 139b is fixed to the machine body by means of the screw portion 21, the detection accuracy of the deterioration of the lubricant 131 a of the machine body can be suppressed from degrading. In other words, in each of the industrial robot 100 and the reducers of the industrial robot 100, in the case where each of the lubricant deterioration sensors such as the lubricant deterioration sensor 139b is fixed to the machine body, the detection accuracy of the deterioration of the lubricant 131 a of the machine body by the lubricant deterioration sensor can be suppressed from degrading.

In each of the lubricant deterioration sensors such as the lubricant deterioration sensor 139b, the screw portion 21 likely deforms toward the inner side thereof when the screw portion 21 is threaded into the screw hole of the machine body and made in contact with the machine body. Thus, in the case of fixing the screw portion 21 to the machine body, the detection accuracy of the deterioration of the lubricant of the machine body can be suppressed efficiently from degrading.

In a case where the housing 20 includes a component other than the screw portion 21 to be fixed to the machine body such as the arm 113, the screw portion 21 shown in Fig. 3 may simply be a contact portion that contacts with the machine body on the outer side thereof. For example, in the housing 20, the screw portion 21 may simply be a cylindrical portion having no screw. In each of the lubricant deterioration sensors such as the lubricant deterioration sensor 139b, in the case where the contact portion acts as a portion to be inserted into the hole of the machine body, the contact portion likely deforms toward the inside when the contact portion is inserted into the hole of the machine body and made in contact with the machine body. Thus, like the configuration where the contact portion is the screw portion 21, in the case where the contact portion contacts with the machine body, the detection accuracy of the deterioration of the lubricant of the machine body can be suppressed efficiently from degrading. In each of the lubricant deterioration sensors such as the lubricant deterioration sensor 139b, even in the configuration that the contact portion is made in contact with the machine body without being inserted into the hole of the machine body, even if the contact portion is made in contact on the outer side thereof with the machine body and is deformed toward the inner side, the deformation of the contact portion is hardly transmitted to the supporting member 30 which is disposed in separation from the contact portion. Thus, in the case where the contact portion contacts with the machine body, the detection accuracy of the deterioration of the lubricant of the machine body can be suppressed from degrading.

To the lubricant 131 a, there is sometimes added various kinds of additive such as friction reducing agent like organic molybdenum such as MoDTC or MoDTP for reducing the friction of a friction surface, extreme-pressure additive such as SP-based additive for improving extreme-pressure lubricity representing the performance for suppressing the sticking of the friction surface, and dispersing agent such as calcium sulfonate for suppressing the generation and adhesion of sludge. These additives are separated from the lubricant 131a in accordance with the deterioration of the lubricant 131 a in such a manner that the additive adheres to, binds with or settles on the metal surface of the industrial robot 100 and the reducer. Each of the lubricant deterioration sensors can specify, based on the detected colors, not only an amount of ion powder within the lubricant 131 a but also a deterioration degree of the base oil and increase of the contaminant such as sludge due to the reduction of various kinds of additive added to the lubricant 131 a. Thus, each of the industrial robot 100 and the reducers of the industrial robot 100 can improve the prediction accuracy of a failure as compared with a technique where a failure of the reducer is predicted only based on a density of iron powder.

It is preferable that the lubricant 131 a does not contain molybdenum such as MoDTC or MoDTP as the additive.

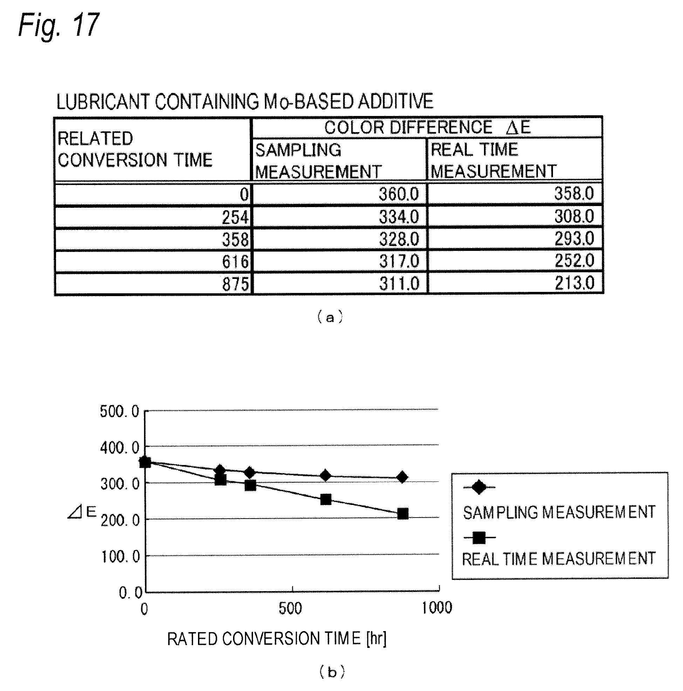

In order to investigate whether or not molybdenum contained in the lubricant 131a as the additive is a cause of the degradation of the performance of the lubricant deterioration sensor, the inventors of the present application have performed the comparative experimentation as to the degradation of the performance of the lubricant deterioration sensor between the lubricant 131 a containing molybdenum as the additive and the lubricant 131 a not containing molybdenum as the additive. In a device for the experimentation prepared so as to have the substantially same configuration as the articular portion 130, under a condition that the maximum output rotation speed was 15 rpm, the maximum load torque was 2.5 times as large as the rated torque of the reducer 131 and a load moment was the rated moment of the reducer 131, this experimentation was performed in a following manner. That is, a reciprocal rotation movement of rotating the supporting body 134 by 45 degrees in a reverse direction with respect to the case 133 after rotating the supporting body by 45 degrees in a forward direction with respect to the case was repeatedly continued. The lubricant used in this experimentation as the lubricant 131a containing molybdenum as the additive contains organic molybdenum as the friction reducing agent, the extreme-pressure additive, the dispersing agent and antioxidant each in a rage of 0.1 % to 10% as the additive. As compared with the lubricant used in this experimentation as the lubricant 131a containing molybdenum as the additive, the lubricant used in this experimentation as the lubricant 131 a not containing molybdenum as the additive differs only in a point that the organic molybdenum as the friction reducing agent is not contained. The results of this experimentation are shown in Figs. 17(a) to 18(b).

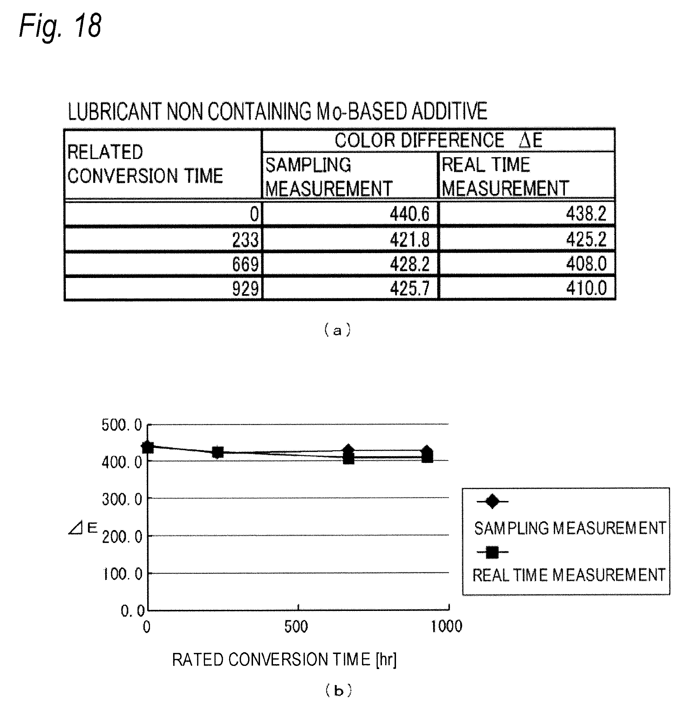

Fig. 17(a) is a table showing the experimentation result of the chorological change of the difference ΔE in a case where the lubricant 131a contains molybdenum as the additive. Fig. 17(b) is a graph of the experimentation result shown in Fig. 17(a). Fig. 18(a) is a table showing the experimentation result of the chorological change of the difference ΔE in a case where the lubricant 131a contains molybdenum as the additive. Fig. 18(b) is a graph of the experimentation result shown in Fig. 18(a).

In Figs. 17(a) to 18(b), a rated conversion time was obtained so that a time period during which the device for the experimentation was actually driven was converted into a time period of a case that the output rotation speed is 15 rpm and the load torque is the rated torque of the reducer 131, based on the output rotation speed and the load torque of the reducer 131 in a case where the device for the experimentation was actually driven. The life time of the reducer 131 is defined as 6,000 hours in a case where the reducer 131 is continuously driven under a condition that the output rotation speed is 15 rpm and the load torque is the rated torque of the reducer 131. Further, the sampling measurement was performed so that the lubricant 131 a was drained from the device for the experimentation each time of the measurement, and a color difference ∆E of the colors of the lubricant 131 a drained from the device for the experimentation with respect to black was measured by using a lubricant deterioration sensor like the lubricant deterioration sensor 139b. Further, the real time measurement was performed so that the color difference ∆E of the colors of the lubricant 131 a within the device for the experimentation with respect to black was measured by using the lubricant deterioration sensor 139b attached to the device for the experimentation.

Since the right-angle prisms of the gap forming member of the lubricant deterioration sensor used at the time of the sampling measurement are made in contact with the lubricant 131a only at the time of the measurement, sludge generated by the deterioration of the lubricant 131 a does not adhere to the prisms. Thus, the experimentation result of the sampling measurement represents the state of the lubricant 131 a quite precisely.

On the other hand, since the right-angle prisms 61, 62 of the gap forming member 60 of the lubricant deterioration sensor 139b used at the time of the real time measurement are always made in contact with the lubricant 131a, sludge generated by the deterioration of the lubricant 131 a adheres to the prisms. Thus, the experimentation result of the real time measurement is influenced by the sludge adhered to the right-angle prisms 61, 62.

Therefore, a difference between the experimentation result of the sampling measurement and the experimentation result of the real time measurement represents a degree of the influence of the sludge adhered to the right-angle prisms 61, 62.

As shown in Figs. 17(a) to 18(b), as compared with the case where the lubricant 131 a contains molybdenum as the additive, when the lubricant 131 a does not contain molybdenum as the additive, the difference between the experimentation result of the sampling measurement and the experimentation result of the real time measurement is small even if the experimentation time periods are substantially same therebetween. In other words, it is clear that this case is less influenced by the sludge adhered to the right-angle prisms 61, 62.

As explained above, when the lubricant 131 a does not contain molybdenum as the additive, each of the industrial robot 100 and the reducers of the industrial robot 100 can prevent a phenomenon that molybdenum adheres to the right-angle prisms of the gap forming member as the sludge. Thus, generation of the adhesion of the sludge to the right-angle prisms of the gap forming member can be suppressed and hence the degradation of the performance of the lubricant deterioration sensor can be suppressed. As a result, each of the industrial robot 100 and the reducers of the industrial robot 100 can prevent the degradation of the performance of the lubricant deterioration sensor which enables to immediately specify the kinds and amounts of the contaminant within the lubricant 131 a.

Further, in each of the lubricant deterioration sensors such as the lubricant deterioration sensor 139b, the housing 20 supports the supporting member 30 so as to be rotatable so that the direction of the opening 60b of the oil gap 60a changes when the supporting member 30 rotates. Thus, in a case where the housing 20 is fixed to the industrial robot 100, the direction of the opening 60b of the oil gap 60a at the time of fixing the housing 20 to the industrial robot 100 can be adjusted so that the detection accuracy of the deterioration of the lubricant 131 a of the industrial robot 100 becomes high. As a result, each of the industrial robot 100 and the reducers of the industrial robot 100 enables to predict a failure with a high accuracy.

Further, in each of the lubricant deterioration sensors such as the lubricant deterioration sensor 139b, the supporting member 30 includes the tool contact portion 51 at a position not contacting with the lubricant 131 a when the housing 20 is fixed to the industrial robot 100. The tool contact portion is a portion which receives the rotational driving force with respect to the housing 20 from the outside by the contact force. Thus, in each of the lubricant deterioration sensors, after the housing 20 is fixed to the industrial robot 100, the direction of the opening 60b of the oil gap 60a at the time of fixing the housing 20 to the industrial robot 100 can be adjusted so that the detection accuracy of the deterioration of the lubricant 131a of the industrial robot 100 becomes high.

Furthermore, in each of the lubricant deterioration sensors such as the lubricant deterioration sensor 139b, each of the bolts 12 with hexagon holes includes the tool contact portion 12a at the position not contacting with the lubricant 131a when the housing 20 is fixed to the industrial robot 100. Each of the bolts with hexagon holes is configured so as to prevent the rotation of the supporting member 30 with respect to the housing 20 by contacting with both the supporting member 30 and the housing 20. The tool contact portion is a portion which receives the driving force for contacting with both the supporting member 30 and the housing 20 from the outside by the contact force. Thus, in each of the lubricant deterioration sensors, after the housing 20 is fixed to the industrial robot 100, the direction of the opening 60b of the oil gap 60a at the time of fixing the housing 20 to the industrial robot 100 can be fixed so that the detection accuracy of the deterioration of the lubricant 131 a of the industrial robot 100 becomes high.

Furthermore, in each of the lubricant deterioration sensors, since the light emitting element is the white LED for emitting white light, the sensor can be miniaturized as compared with a configuration where the light emitting element is a lamp other than an LED, for example. Thus, each of the industrial robot 100 and the reducers of the industrial robot 100 can be miniaturized. The light emitting element according to this invention may be one other than the white LED. For example, the light emitting element may be a lamp other than an LED. Further, the light emitting element may be configured to include a red LED or a red lamp other than an LED, a green LED or a green lamp other than an LED and a blue LED or a blue lamp other than an LED, to thereby emit white light by composing light of respective colors emitted from these LEDs or these lamps other than LEDs.

Furthermore, in each of the lubricant deterioration sensors, the gap forming member 60 is provided with the light reflection surfaces 61b, 62b for bending the optical path 10a. Thus, as compared with the configuration where the optical path 10a from the white LED 72 to the RGB sensor 73 is straight, the entire configuration can be miniaturized by disposing the white LED 72 and the RGB sensor 73 close to each other. Furthermore, in each of the lubricant deterioration sensors, the gap forming member 60 has a function of bending the optical path 10a as well as a function of forming the oil gap 60a. Thus, the number of the components can be reduced as compared with a configuration where a member for bending the optical path 10a is separately provided in place of the gap forming member 60. As a result, each of the industrial robot 100 and the reducers of the industrial robot 100 can be miniaturized and also reduce the number of the components.

In particular, each of the lubricant deterioration sensors is configured so that the gap forming member 60 is formed by the two right-angle prisms 61, 62 respectively provided with the light reflection surfaces 61 b, 62b each for bending the optical path 10a by 90 degrees, and that the optical path 10a is bent by 180 degrees by the light reflection surfaces 61 b, 62b of the two right-angle prisms 61, 62 and the oil gap 60a is formed between the two right-angle prisms 61, 62. Thus, the sensor can be miniaturized with the simple configuration having a small number of the components. As a result, each of the industrial robot 100 and the reducers of the industrial robot 100 can be miniaturized with the simple configuration having the small number of the components.

Furthermore, each of the lubricant deterioration sensors is configured so that the sensor has the holder 40 surrounding at least a part of the optical path 10a and the surface of the holder 40 is treated to the treatment for preventing light reflection. Thus, the RGB sensor 73 can be prevented from receiving unnecessary reflection light. As a result, as compared with a configuration where the RGB sensor 73 receives unnecessary reflection light, each of the lubricant deterioration sensors can improve the detection accuracy of the colors of the contaminant within the lubricant 131 a. Accordingly, each of the industrial robot 100 and the reducers of the industrial robot 100 can improve the prediction accuracy of a failure.

Furthermore, in each of the lubricant deterioration sensors, an oil repelling treatment may be performed on the surface planes of the gap forming member 60 forming the oil gap 60a, that is, the light emission surface 61c of the right-angle prism 61 and the light incident surface 62a of the right-angle prism 62. In each of the lubricant deterioration sensors, when each of the light emission surface 61c of the right-angle prism 61 and the light incident surface 62a of the right-angle prism 62 is treated to the oil repelling treatment, the lubricant 131a flows through the oil gap 60a easily. Thus, as compared with a configuration where the lubricant 131 a likely remains at the oil gap 60a, the detection accuracy of the colors of the contaminant within the lubricant 131a can be improved. Furthermore, in each of the lubricant deterioration sensors, when each of the light emission surface 61 c of the right-angle prism 61 and the light incident surface 62a of the right-angle prism 62 is treated to the oil repelling treatment, dirt is unlikely adhered to each of the light emission surface 61c of the right-angle prism 61 and the light incident surface 62a of the right-angle prism 62. Thus, the degradation of the detection accuracy of the colors of the contaminant within the lubricant 131a due to the adhesion of dirt can be suppressed. Accordingly, each of the industrial robot 100 and the reducers of the industrial robot 100 can improve the prediction accuracy of a failure.

The prediction accuracy of a failure of the reducer can be improved by jointly using the lubricant deterioration sensor according to this invention, the temperature sensor for measuring the temperature of the lubricant and a monitoring mechanism for a current value of the motor etc.

Although each of the right-angle prisms 61, 62 of the gap forming member 60 is made of glass in this embodiment, each of them may be formed by material other than glass such as silicon resin. When each of the prisms 61, 62 is formed by silicon resin, dirt can be unlikely adhered to the surface planes thereof forming the oil gap 60a.

Although the gap forming member 60 is configured by the two right-angle prisms 61, 62 in this embodiment, the gap forming member may be configured by three or more prisms.

In each of the lubricant deterioration sensors, the white LED 72 and the RGB sensor 73 may be configured in an arrangement other than that explained in this embodiment. For example, in each of the lubricant deterioration sensors, the optical path 10a from the white LED 72 to the RGB sensor 73 may be straight

Further, in each of the lubricant deterioration sensors, the optical path 10a may be bent by employing a configuration other than the right-angle prisms.

In each of the lubricant deterioration sensors, for example, a battery such as a battery cell may be used as an electric power supply means, and a wireless communication may be employed as a means for outputting the detection result to the external device.

The configuration of an industrial robot as a machine according to the second embodiment will be explained.

The configuration of the industrial robot according to this embodiment is same as that of the industrial robot 100 (see Fig. 1) according to the first embodiment except for configuration explained bellow. Thus, of the configuration of the industrial robot according to this embodiment, components same as those of the industrial robot 100 are referred to by the same symbols as those of the industrial robot 100, with detailed explanation thereof being omitted.

Of the industrial robot according to this embodiment, portions except for lubricant such as lubricant 231a described later, lubricant deterioration sensors such as lubricant deterioration sensors 237, 239 described later and cleaning members described later constitute the machine body of this embodiment in a case where the industrial robot is the machine according to this invention.

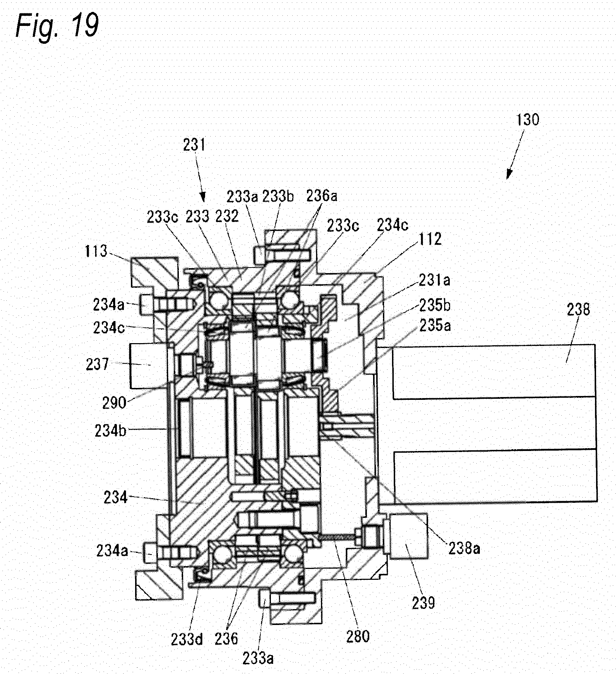

Fig. 19 is a sectional diagram of the articular portion 130 of the industrial robot according to this embodiment. Although the explanation is made hereinafter as to the articular portion 130, the configuration of each of the articular portions 120, 140 to 170 is substantially same.

As shown in Fig. 19, the articular portion 130 includes a reducer 231 for connecting between the arm 112 and the arm 113, a motor 238 provided with a gear 238a at the output shaft thereof and fixed to the arm 112 by means of not-shown bolts, and a lubricant deterioration sensor 239 for detecting the deterioration of lubricant 231 a for reducing friction generated at the movable portions of the reducer 231. The lubricant 231a is similar to the lubricant 131a according to the first embodiment.

The reducer 231 includes a reducer body 232 and a lubricant deterioration sensor 237 for detecting the deterioration of the lubricant 231a of the reducer body 232. The reducer body 232 constitutes the machine body according to this invention in a case where the reducer 231 is the machine according to this invention.

The reducer body 232 includes a case 233 fixed to the arm 112 by means of bolts 233a, a supporting body 234 fixed to the arm 113 by means of bolts 234a, three gears 235a which are disposed around the center axis of the reducer 231 with a constant interval therebetween and mesh with the gear 238a of the motor 238, three crank shafts 235b which are disposed around the center axis of the reducer 231 with a constant interval therebetween and fixed to the gears 235a, respectively, and two external gears 236 which mesh with internal gears 233b provided at the case 233.

The supporting body 234 is rotatably supported by the case 233 via two bearings 233c. The supporting body 234 is provided with a seal member 234b for preventing leakage of the lubricant 231a. A seal member 233d for preventing leakage of the lubricant 231 a is provided between the case 233 and the supporting body 234. Cleaning members 280 for cleaning the lubricant deterioration sensor 239 are fixed to the supporting body 234.

Each of the crank shafts 235b is rotatably supported by the supporting body 234 via two bearings 234c and further rotatably supported by the external gears 236 via bearings 236a. A cleaning member 290 for cleaning the lubricant deterioration sensor 237 is fixed to the crank shaft 235b.

The lubricant deterioration sensor 237 is fixed to the support body 234. The lubricant deterioration sensor 239 is fixed to the arm 112.

The configuration of each of the lubricant deterioration sensors 237, 239 is same as that of the lubricant deterioration sensor 139b (see Figs. 3 to 5(b)) according to the first embodiment. Thus, of the configuration of each of the lubricant deterioration sensors 237, 239, components same as those of the lubricant deterioration sensor 139b are referred to by the same symbols as those of the lubricant deterioration sensor 139b, with detailed explanation thereof being omitted.

The screw hole of the arm 112, to which the lubricant deterioration sensor 239 is attached, may be used for supplying the lubricant 231 a to the reducer 231 and disposing the lubricant 231 a from the reducer 231 in a state that the lubricant deterioration sensor 239 is detached.

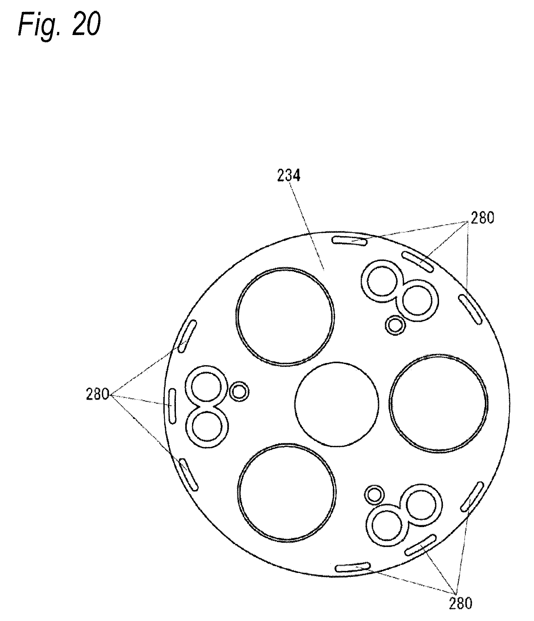

Fig. 20 is a plan view of a component of the supporting body 234 to which cleaning members 280 are fixed.

As shown in Figs. 19 and 20, each of the cleaning members 280 is disposed at a position contacting with both the light emission surface 61c of the right-angle prism 61 and the light incident surface 62a of the right-angle prism 62 in a case where the arm 112 and the supporting body 234 move relatively. In this respect, this arm acts as a sensor side portion of this invention, at which the lubricant deterioration sensor 239 is mounted, and this supporting body acts as a cleaning member side portion of this invention, at which the cleaning members 280 are mounted. The cleaning members 280 are disposed so that three sets each configured by the three cleaning members are disposed around the center axis of the reducer 231 with a constant interval therebetween. Thus, the nine cleaning members in total are disposed around the center axis of the reducer 231.

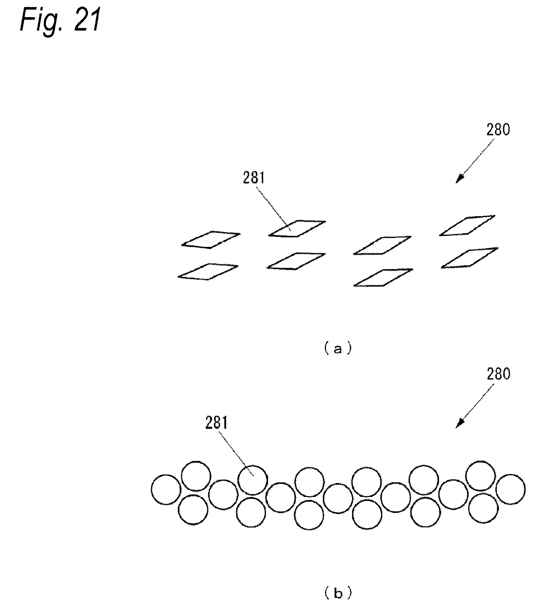

Fig. 21(a) is a plan view of an example of the cleaning member 280. Fig. 21(b) is a plan view of another example of the cleaning member 280 different from that shown in Fig. 21 (a).

As shown in Figs. 21(a) and 21(b), various configurations can be employed as the shape of the cleaning member 280. Each of the cleaning members 280 has a plurality of insertion portions 281 each of which has elasticity and is inserted into the oil gap 60a of the lubricant deterioration sensor 239.

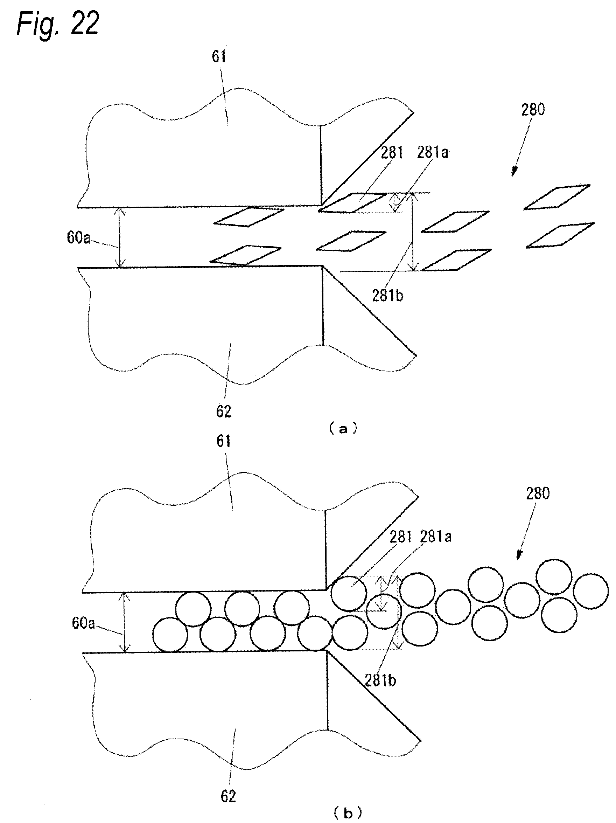

Fig. 22(a) is a plan view of the cleaning member 280 in a case where a part of the cleaning member 280 shown in Fig. 21 (a) is inserted in the oil gap 60a. Fig. 22(b) is a plan view of the cleaning member 280 in a case where a part of the cleaning member 280 shown in Fig. 21 (b) is inserted in the oil gap 60a.

As shown in Figs. 22(a) and 22(b), the width 281 a of each of the insertion portions 281 in the distance direction of the oil gap 60a of the lubricant deterioration sensor 239 is smaller than the distance of the oil gap 60a. Further, the entire width 281 b of the plurality of insertion portions 281 in the distance direction of the oil gap 60a is larger than the distance of the oil gap 60a.

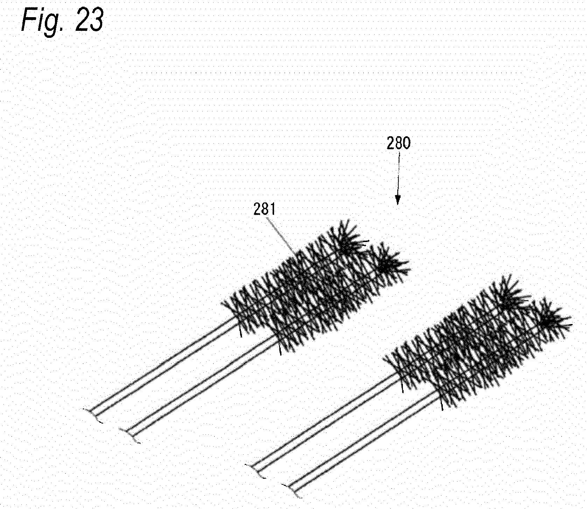

Fig. 23 is a perspective view of the cleaning member 280 in a case that the insertion portion 281 is a brush.

The insertion portion 281 may be a wiper made of rubber or a brush as shown in Fig. 23, for example.

Although the explanation is made above as to the cleaning member 280, the configuration of the cleaning member 290 is substantially same. Each of the cleaning members 290 is disposed at a position contacting with the gap forming surfaces of the lubricant deterioration sensor 237 in a case where the supporting body 234 and the crank shaft 235b move relatively. In this respect, this supporting body acts as the sensor side portion of this invention at which the lubricant deterioration sensor 237 is mounted, and this crank shaft acts as the cleaning member side portion of this invention at which the cleaning members 290 are mounted.

Next, the operation of the industrial robot according to this embodiment will be explained.

First, the operation of the articular portion 130 will be explained. Although the explanation is made hereinafter as to the articular portion 130, the configuration of each of the articular portions 120, 140 to 170 is substantially same.

When the output shaft of the motor 238 of the articular portion 130 rotates, the rotation speed of the motor 238 is reduced by the reducer 231, whereby the arm 113 fixed to the supporting body 234 of the reducer 231 is moved by the rotation force of the motor with respect to the arm 112 fixed to the case 233 of the reducer 231.

In this case, in accordance with the relative movement between the arm 112 and the supporting body 234 of the reducer 231, the cleaning member 280 provided at the supporting body 234 is inserted in the oil gap 60a of the lubricant deterioration sensor 239 provided at the arm 112, as shown in Fig. 22. Then, by the cleaning member 280 inserted in the oil gap 60a of the lubricant deterioration sensor 239, dirt such as sludge adhered to the gap forming surfaces of the lubricant deterioration sensor 239, that is, the light emission surface 61 c of the right-angle prism 61 and the light incident surface 62a of the right-angle prism 62 is rubbed off.

Similarly, in accordance with the relative movement between the supporting body 234 and the crank shaft 235b of the reducer 231, the cleaning member 290 provided at the crank shaft 235b is inserted in the oil gap of the lubricant deterioration sensor 237 provided at the supporting body 234. Then, by the cleaning member 290 inserted in the oil gap of the lubricant deterioration sensor 237, dirt such as sludge adhered to the gap forming surfaces of the lubricant deterioration sensor 237 is rubbed off.

Next, explanation will be made as to the effects of the industrial robot according to this embodiment, each of the reducers of the industrial robot, and each of the lubricant deterioration sensors such as the lubricant deterioration sensor 239.

Of the effects of the industrial robot according to this embodiment, each of the reducers of the industrial robot, and each of the lubricant deterioration sensors such as the lubricant deterioration sensor 239, the explanation will be omitted as to the effects similar to those of the industrial robot 100 according to the first embodiment, each of the reducers of the industrial robot 100, and each of the lubricant deterioration sensors such as the lubricant deterioration sensor 139b

Further, in the industrial robot according to this embodiment, each of the cleaning members 280 is disposed at a position contacting with the gap forming surfaces of the lubricant deterioration sensor 239, that is, both the light emission surface 61c of the right-angle prism 61 and the light incident surface 62a of the right-angle prism 62 in a case where the reducer 231 rotates. Thus, the cleaning member 280 can rub off dirt such as sludge adhered to the gap forming surfaces of the lubricant deterioration sensor 239 each time the arm 112 is driven by the reducer 231. In the industrial robot according to this embodiment, when the arm 112 as the sensor side portion and the supporting body 234 as the cleaning member side portion move relatively, the cleaning member 280 rubs off dirt such as sludge adhered to the gap forming surfaces of the gap forming member 60 of the lubricant deterioration sensor 239. Thus, the degradation of the performance of the lubricant deterioration sensor 239 can be suppressed. Therefore, the industrial robot according to this embodiment can suppress the degradation of the performance of the lubricant deterioration sensor 239 which enables to immediately specify the kinds and amounts of contaminant within the lubricant 231 a.

In the similar manner, in each of the industrial robot and the reducers of the industrial robot according to this embodiment, each of the cleaning members 290 is disposed at a position contacting with the gap forming surfaces of the lubricant deterioration sensor 237 in a case where the reducer 231 rotates. Thus, the cleaning member 290 can rub off dirt such as sludge adhered to the gap forming surfaces of the lubricant deterioration sensor 237 each time the reducer 231 rotates. In the industrial robot according to this embodiment and in each of the reducers of the industrial robot according to this embodiment, in a case where the supporting body 234 as the sensor side portion and the crank shaft 235b as the cleaning member side portion move relatively, the cleaning member 290 rubs off dirt such as sludge adhered to the gap forming surfaces of the lubricant deterioration sensor 237. Thus, the degradation of performance of the lubricant deterioration sensor 237 can be suppressed. Accordingly, each of the industrial robot according to this embodiment and the reducers of the industrial robot according to this embodiment can suppress the degradation of performance of the lubricant deterioration sensor 237 which enables to immediately specify the kinds and amounts of contaminant within the lubricant 231 a.

Further, as described above, the industrial robot according to this embodiment can suppress the degradation of performance of the lubricant deterioration sensors 237 and 239 each of which enables to immediately specify the kinds and amounts of contaminant within the lubricant 231 a. Thus, the accuracy of immediate prediction of a failure can be maintained for a long time.

Further, as described above, the reducer for the industrial robot, that is, each of the reducers for the industrial robot according to this embodiment can suppress the degradation of performance of the lubricant deterioration sensors 237 and 239 each of which enables to immediately specify the kinds and amounts of contaminant within the lubricant 231 a. Thus, the accuracy of immediate prediction of a failure can be maintained for a long time.

As described above, the width 281 a of each of the insertion portions 281 of the cleaning member 280 in the distance direction of the oil gap 60a of the lubricant deterioration sensor 239 is smaller than the distance of the oil gap 60a. Further, the entire width 281 b of the plurality of insertion portions 281 of the cleaning member 280 in the distance direction of the oil gap 60a is larger than the distance of the oil gap 60a. Thus, in the industrial robot according to this embodiment, as compared with a case where the cleaning member 280 is configured only by a single insertion portion which width in the distance direction of the oil gap 60a is larger than the distance of the oil gap 60a, the pressure applied to the lubricant deterioration sensor 239 by the cleaning member 280 at the time of inserting the cleaning member 280 in the oil gap 60a can be mad small. As a result, the degradation of performance of the lubricant deterioration sensor 239 due to the pressure applied to the lubricant deterioration sensor 239 from the cleaning member 280 can be suppressed. Further, in the industrial robot according to this embodiment, dirt such as sludge adhered to the gap forming surfaces of the gap forming member 60 of the lubricant deterioration sensor 239, that is, both the light emission surface 61c of the right-angle prism 61 and the light incident surface 62a of the right-angle prism 62 can be rubbed off by the plurality of insertion portions 281 of the cleaning member 280.

Similarly, the width of each of the insertion portions of the cleaning member 290 in the distance direction of the oil gap of the lubricant deterioration sensor 237 is smaller than the distance of the oil gap of the lubricant deterioration sensor 237. Further, the entire width of the plurality of insertion portions of the cleaning member 290 in the distance direction of the oil gap of the lubricant deterioration sensor 237 is larger than the distance of the oil gap of the lubricant deterioration sensor 237. Thus, in the industrial robot according to this embodiment and in each of the reducers of the industrial robot according to this embodiment, as compared with a case where the cleaning member 290 is configured only by a single insertion portion which width in the distance direction of the oil gap of the lubricant deterioration sensor 237 is larger than the distance of the oil gap of the lubricant deterioration sensor 237, the pressure applied to the lubricant deterioration sensor 237 by the cleaning member 290 at the time of inserting the cleaning member 290 in the oil gap of the lubricant deterioration sensor 237 can be mad small. As a result, the degradation of performance of the lubricant deterioration sensor 237 due to the pressure applied to the lubricant deterioration sensor 237 from the cleaning member 290 can be suppressed. Further, in each of the industrial robot according to this embodiment and the reducers of the industrial robot according to this embodiment, dirt such as sludge adhered to the gap forming surfaces of the gap forming member of the lubricant deterioration sensor 237 can be effectively rubbed off by the plurality of insertion portions of the cleaning member 290.

The lubricant deterioration sensor 239 is provided at the outside thereof with the cleaning members 280 for rubbing off dirt such as sludge adhered to the gap forming surfaces. Thus, the configuration of this sensor can be miniaturized as compared with a case where the lubricant deterioration sensor itself is provided with a configuration for rubbing off dirt such as sludge adhered to the gap forming surfaces. Although the explanation is made as to the lubricant deterioration sensor 239, the aforesaid explanation is substantially applied to the lubricant deterioration sensor 237.

In each of the lubricant deterioration sensors such as the lubricant deterioration sensor 239, the housing 20 supports the supporting member 30 so as to be rotatable so that the direction of the opening 60b of the oil gap 60a changes when the supporting member 30 rotates. Thus, the direction of the opening 60b of the oil gap 60a in the case of fixing the housing 20 to the industrial robot can be adjusted so that the dirt cleaning effects of the cleaning members 280, 290 in the case of fixing the housing 20 to the industrial robot can be enhanced. As a result, each of the industrial robot according to this embodiment and the reducers of the industrial robot according to this embodiment enables to predict a failure with a high accuracy.

Further, in each of the lubricant deterioration sensors such as the lubricant deterioration sensor 239, the supporting member 30 includes the tool contact portion 51 at a position not contacting with the lubricant 231 a when the housing 20 is fixed to the industrial robot according to this embodiment. This tool contact portion acts as the portion for receiving the rotational driving force with respect to the housing 20 from the outside by the contact force. Thus, in each of the lubricant deterioration sensors, the direction of the opening 60b of the oil gap 60a in the case of fixing the housing 20 to the industrial robot according to this embodiment can be adjusted so that the dirt cleaning effects of the cleaning members 280, 290 can be enhanced after fixing the housing 20 to the industrial robot according to this embodiment.

Furthermore, in each of the lubricant deterioration sensors such as the lubricant deterioration sensor 239, each of the bolts 12 with hexagon holes includes the tool contact portion 12a at the position not contacting with the lubricant 231 a when the housing 20 is fixed to the industrial robot according to this embodiment. In this respect, each of these bolts with hexagon holes is configured so as to prevent the rotation of the supporting member 30 with respect to the housing 20 by contacting with both the supporting member 30 and the housing 20. This tool contact portion acts as the portion for receiving the driving force for contacting with both the supporting member 30 and the housing 20 from the outside by the contact force. Thus, in each of the lubricant deterioration sensors, the direction of the opening 60b of the oil gap 60a in the case of fixing the housing 20 to the industrial robot according to this embodiment can be fixed so that the dirt cleaning effects of the cleaning members 280, 290 can be enhanced after fixing the housing 20 to the industrial robot according to this embodiment.

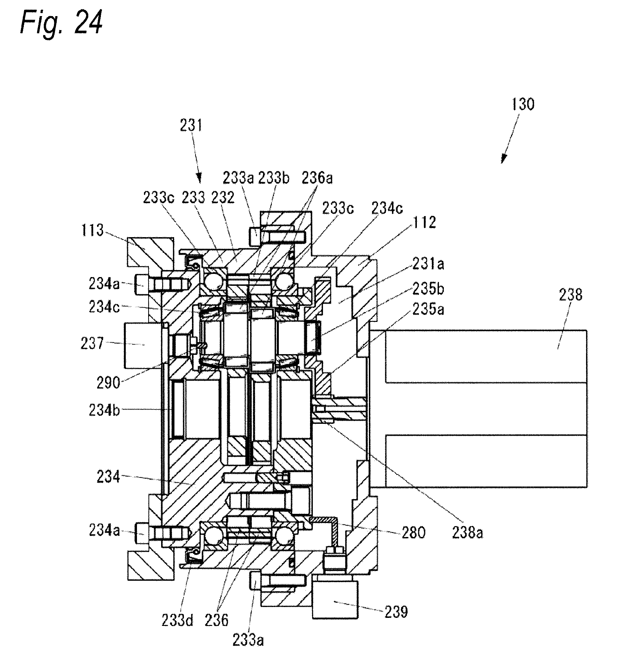

As shown in Fig. 19, the lubricant deterioration sensor 237 is disposed with respect to the cleaning member 290 in the extending direction of the rotation shaft of the cleaning member 290. Similarly, as shown in Fig. 19, the lubricant deterioration sensor 239 is disposed with respect to the cleaning member 280 in the extending direction of the rotation shaft of the cleaning member 280. However, the arrangement between the lubricant deterioration sensor and the cleaning member may not be limited thereto. For example, as shown in Fig. 24, the lubricant deterioration sensor 239 may be disposed with respect to the cleaning member 280 in a direction orthogonal to the extending direction of the rotation shaft of the cleaning member 280.

Further, the mounting position of each of the lubricant deterioration sensors is not limited to that shown in this embodiment, and preferably may be set suitably according to the usage etc. of the industrial robot.

Although the machine according to this invention is the reducer for the industrial robot or the industrial robot in each of the aforesaid embodiments, the machine may be a machine other than the reducer for the industrial robot or the industrial robot.

The present application is based on Japanese Patent Application (Japanese Patent Application No. 2012-032739) filed on February 17, 2012.

Industrial Applicability

According to this invention, the lubricant deterioration sensor can be provided which enables to immediately specify the kinds and amounts of the contaminant within the lubricant of the machine.

Reference Signs List

| | |
|---|---|
| 10a | optical path |
| 12 | bolt with hexagon hole (rotation preventing member) |
| 12a | tool contact portion (contact driving force receiving portion) |
| 20 | housing (fixing member) |
| 21 | screw portion (contact portion) |
| 30 | supporting member |
| 51 | tool contact portion (rotational driving force receiving portion) |
| 60 | gap forming member |
| 60a | oil gap |
| 60b | opening |
| 72 | white LED (light emitting element) |
| 73 | RGB sensor (color light reception element) |
| 100 | industrial robot (machine) |
| 112 | arm (sensor side portion) |
| 113 - 116 | arm |
| 113a | screw hole |
| 131 | reducer (reducer for industrial robot, machine) |
| 131a | lubricant |
| 132 | reducer body (machine body) |
| 137a, 137b, 139a, 139b | lubricant deterioration sensor |
| 231 | reducer (reducer for industrial robot, machine) |
| 231a | lubricant |
| 232 | reducer body (machine body) |
| 234 | supporting body (sensor side portion, cleaning member side portion) |
| 235b | crank shaft (cleaning member side portion) |
| 237, 239 | lubricant deterioration sensor |
| 280, 290 | cleaning member |
| 281 | insertion portion |

**Claims**

1. A lubricant deterioration sensor (137a, 137b, 139a, 139b, 237, 239) for detecting deterioration of lubricant (131 a) of a machine body in a state where the lubricant deterioration sensor (137a, 137b, 139a, 139b, 237, 239) is mounted in the machine body, the lubricant deterioration sensor (137a, 137b, 139a, 139b, 237, 239) comprising:

   a light emitting element (72) configured to emit light;
   a color light reception element (73) configured to detect color of received light;
   a gap forming member (60) forming an oil gap (60a) into which the lubricant (131 a) enters;
   a supporting member (30) supporting the light emitting element (72), the color light reception element (73) and the gap forming member (60); and
   a fixing member (20) configured to be fixed to the machine body,
   wherein
   the gap forming member (60) is configured to transmit the light emitted from the light emitting element (72),
   the oil gap (60a) is disposed on an optical path (10a) from the light emitting element (72) to the color light reception element (73),
   the fixing member (20) includes a contact portion (21) that is configured to contact with the machine body on an outer side of the contact portion (21), and
   the lubricant deterioration sensor being **characterized in that** the supporting member (30) is disposed on an inner side of the contact portion (21) so as to be separated from the contact portion (21) by a gap (10b) so that at least a part of the optical path (10a) is disposed on the inner side of the contact portion (21).

2. The lubricant deterioration sensor (137a, 137b, 139a, 139b, 237, 239) according to claim 1, wherein the contact portion (21) is configured to be inserted into a hole (113a) of the machine body.

3. The lubricant deterioration sensor (137a, 137b, 139a, 139b, 237, 239) according to claim 2, wherein the hole (113a) is a screw hole, and the contact portion (21) is a screw portion which is configured to be inserted into the screw hole (113a) of the machine body and fixed thereto.

4. The lubricant deterioration sensor (137a, 137b, 139a, 139b, 237, 239) according to one of claims 1 to 3, wherein the fixing member (20) rotatably supports the supporting member (30) so that a direction of an opening (60b) of the oil gap (60a) changes when the supporting member (30) rotates.

5. The lubricant deterioration sensor (137a, 137b, 139a, 139b, 237, 239) according to claim 4, wherein the supporting member (30) includes a rotational driving force receiving portion (51), which receives a rotational driving force with respect to the fixing member (20) from outside by a contact force, at a position where the rotational driving force receiving portion (51) does not contact with the lubricant (131 a) in a state where the fixing member (20) is fixed to the machine body.

6. The lubricant deterioration sensor (137a, 137b, 139a, 139b, 237, 239) according to claim 4 or 5, further comprising:

   a rotation preventing member (12) contacting with both the supporting member (30) and the fixing member (20) so as to prevent a rotation of the supporting member (30) with respect to the fixing member (20), wherein the rotation preventing member (12) includes a contact driving force receiving portion (12a), which receives a driving force for contacting with both the supporting member (30) and the fixing member (20) from outside by a contact force, at a position where the contact driving force receiving portion (12a) does not contact with the lubricant (131 a) in a state where the fixing member (20) is fixed to the machine body.

7. A machine, comprising:

   the lubricant deterioration sensor (137a, 137b, 139a, 139b, 237, 239) according to one of claims 1 to 6; and the machine body.

8. The machine according to claim 7, further comprising:

   a cleaning member (280, 290) configured to clean the lubricant deterioration sensor (137a, 137b, 139a, 139b, 237, 239), wherein

the machine body includes a sensor side portion (112, 234) at which the lubricant deterioration sensor (137a, 137b, 139a, 139b, 237, 239) is mounted and a cleaning member side portion (234, 235b) which is movable with respect to the sensor side portion (112, 234) and at which the cleaning member (280, 290) is mounted, and wherein

the cleaning member (280, 290) is disposed at a position contacting with gap forming surfaces of the gap forming member (60) forming the oil gap (60a) when the sensor side portion (112, 234) and the cleaning member side portion (234, 235b) move relatively.

9. The machine according to claim 8, wherein
the cleaning member (280, 290) includes a plurality of insertion portions (281) configured to be inserted in the oil gap (60a) and having elasticity,
a width of each of the insertion portions (281) in a distance direction of the oil gap (60a) is smaller than the distance of the oil gap (60a), and
an entire width of the plurality of insertion portions (281) in the distance direction of the oil gap (60a) is larger than the distance of the oil gap (60a).

10. The machine according to one of claims 7 to 9, wherein
the machine is a reducer (131, 231) for an industrial robot (100), and
the machine body is a main body of the reducer (131, 231).

11. The machine according to one of claims 7 to 9, wherein
the machine is an industrial robot (100),
the machine body includes an arm (112) and a reducer (131, 231) used at an articular portion (130) of the arm (112), and
the lubricant (131a) is lubricant for the reducer (131, 231).

12. The machine according to claim 8 or 9, wherein
the machine is an industrial robot (100),
the machine body includes an arm (112) and a reducer (131, 231) used at an articular portion (130) of the arm (112),
the lubricant (131 a) is lubricant for the reducer (131, 231),
the arm (112) is the sensor side portion,
the reducer (131, 231) is the cleaning member side portion, and
the cleaning member (280, 290) is disposed at a position contacting with the gap forming surfaces when the reducer (131, 231) rotates.

**Patentansprüche**

1. Sensor (137a, 137b, 139a, 139b, 237, 239) für Alterung von Schmiermittel, mit dem Alterung von Schmiermittel (131a) eines Maschinen-Körpers in einem Zustand erfasst wird, in dem der Sensor (137a, 137b, 139a, 139b, 237, 239) für Alterung von Schmiermittel in dem Maschinen-Körper installiert ist, wobei der Sensor (137a, 137b, 139a, 139b, 237, 239) für Alterung von Schmiermittel umfasst:

ein lichtemittierendes Element (72), das so ausgeführt ist, dass es Licht emittiert;
ein Farblicht-Empfangselement (73), das so ausgeführt ist, dass es die Farbe von empfangenem Licht erfasst;
ein Teil (60) zum Bilden eines Spaltes, das einen Öl-Spalt (60a) bildet, in den das Schmiermittel (131 a) eintritt;
ein Trageteil (30), das das lichtemittierende Element (72), das Farblicht-Empfangselement (73) und das Teil (60) zum Bilden eines Spaltes trägt; und
ein Befestigungselement (20), das so ausgeführt ist, dass es an dem Maschinen-Körper befestigt wird,
wobei
das Teil (60) zum Bilden eines Spaltes so ausgeführt ist, dass es das von dem lichtemittierenden Element (72) emittierte Licht durchlässt,
der Öl-Spalt (60a) auf einem Lichtweg (10a) von dem lichtemittierenden Element (72) zu dem Farblicht-Empfangselement (73) angeordnet ist,
das Befestigungselement (20) einen Kontaktabschnitt (21) enthält, der so ausgeführt ist, dass er mit dem Maschinen-Körper an einer Außenseite des Kontaktabschnitts (21) in Kontakt ist, und
der Sensor für Alterung von Schmiermittel **dadurch gekennzeichnet ist, dass** das Trageteil (30) an einer Innenseite des Kontaktabschnitts (21) so angeordnet ist, dass es von dem Kontaktabschnitt (21) durch einen Spalt (10b) so getrennt ist, dass wenigstens ein Teil des Lichtweges (10a) an der Innenseite des Kontaktabschnitts (21) angeordnet ist.

2. Sensor (137a, 137b, 139a, 139b, 237, 239) für Alterung von Schmiermittel nach Anspruch 1, wobei der Kontaktabschnitt (21) so ausgeführt ist, dass er in ein Loch (113a) des Maschinen-Körpers eingeführt wird.

3. Sensor (137a, 137b, 139a, 139b, 237, 239) für Alterung von Schmiermittel nach Anspruch 2, wobei
das Loch (113a) ein Schraubloch ist, und
der Kontaktabschnitt (21) ein Schraubabschnitt ist, der so ausgeführt ist, dass er in das Schraubloch (113a) des Maschinen-Körpers eingeführt und daran befestigt wird.

4. Sensor (137a, 137b, 139a, 139b, 237, 239) für Alterung von Schmiermittel nach einem der Ansprüche 1 bis 3, wobei
das Befestigungsteil (20) das Trageteil (30) drehbar so trägt, dass sich eine Richtung einer Öffnung (60b) des Öl-Spaltes (60a) ändert, wenn sich das Trageteil (30) dreht.

5. Sensor (137a, 137b, 139a, 139b, 237, 239) für Alterung von Schmiermittel nach Anspruch 4, wobei
das Trageteil (30) einen Abschnitt (51) zum Aufnehmen von Dreh-Antriebskraft, der eine Dreh-Antriebskraft in Bezug auf das Befestigungsteil (20) von außen über eine Kontaktkraft aufnimmt, an einer Position enthält, an der der Abschnitt (51) zum Aufnehmen von Dreh-Antriebskraft in einem Zustand, in dem das Befestigungsteil (20) an dem Maschinen-Körper befestigt ist, nicht mit dem Schmiermittel (131 a) in Kontakt kommt.

6. Sensor (137a, 137b, 139a, 139b, 237, 239) für Alterung von Schmiermittel nach Anspruch 4 oder 5, der des Weiteren umfasst:

   ein Teil (12) zum Verhindern von Drehung, das sowohl mit dem Trageteil(30) als auch dem Befestigungsteil (20) in Kontakt ist, um eine Drehung des Trageteils (30) in Bezug auf das Befestigungsteil (20) zu verhindern, wobei
   das Teil (12) zum Verhindern von Drehung einen Abschnitt (12a) zum Aufnehmen von Kontakt-Antriebskraft, der eine Kontakt-Antriebskraft zum Herstellen von Kontakt sowohl mit dem Trageteil (30) als auch dem Befestigungsteil (20) von außen über eine Kontaktkraft aufnimmt, an einer Position enthält, an der der Abschnitt (12a) zum Aufnehmen von Kontakt-Antriebskraft in einem Zustand, in dem das Befestigungsteil (20) an dem Maschinen-Körper befestigt ist, nicht mit dem Schmiermittel (131a) in Kontakt kommt.

7. Maschine, die umfasst:

   den Sensor (137a, 137b, 139a, 139b, 237, 239) für Alterung von Schmiermittel nach einem der Ansprüche 1 bis 6; und
   den Maschinen-Körper.

8. Maschine nach Anspruch 7, die des Weiteren umfasst:

   ein Reinigungselement (280, 290), das so ausgeführt ist, dass es den Sensor (137a, 137b, 139a, 139b, 237, 239) für Alterung von Schmiermittel reinigt, wobei
   der Maschinen-Körper einen Abschnitt (112, 234) an der Seite des Sensors, an dem der Sensor (137a, 137b, 139a, 139b, 237, 239) für Alterung von Schmiermittel installiert ist, sowie einen Abschnitt (234, 235b) an der Seite des Reinigungselementes enthält, der in Bezug auf den Abschnitt (112, 234) an der Seite des Sensors bewegt werden kann und an dem das Reinigungselement (280, 290) installiert ist, und wobei
   das Reinigungselement (280, 290) an einer Position angeordnet ist, an der es mit den den Spalt bildenden Flächen des Teils (60) zum Bilden eines Spaltes, die den Öl-Spalt (60a) bilden, in Kontakt ist, wenn sich der Abschnitt (112, 234) an der Seite des Sensors und der Abschnitt (234, 235b) an der Seite des Reinigungselementes relativ zueinander bewegen.

9. Maschine nach Anspruch 8, wobei
das Reinigungselement (280, 290) eine Vielzahl von Einführungsabschnitten (281) enthält, die so ausgeführt sind, dass sie in den Öl-Spalt (60a) eingeführt werden und Elastizität aufweisen,
eine Breite jedes der Einführungsabschnitte (281) in einer Zwischenraum-Richtung des Öl-Spaltes (60a) kleiner ist als der Zwischenraum des Öl-Spaltes (60a), und

eine gesamte Breite der Vielzahl von Einführungsabschnitten (281) in der Zwischenraum-Richtung des Öl-Spaltes (60a) größer ist als der Zwischenraum des Öl-Spaltes (60a).

10. Maschine nach einem der Ansprüche 7 bis 9, wobei
die Maschine ein Untersetzungsgetriebe (131, 231) für einen Industrieroboter (100) ist, und
der Maschinen-Körper ein Hauptkörper des Untersetzungsgetriebes (131, 231) ist.

11. Maschine nach einem der Ansprüche 7 bis 9, wobei
die Maschine ein Industrieroboter (100) ist,
der Maschinen-Körper einen Arm (112) sowie ein Untersetzungsgetriebe (131, 231) einschließt, das an einem Gelenkabschnitt (130) des Arms (112) eingesetzt wird, und das Schmiermittel (131 a) ein Schmiermittel für das Untersetzungsgetriebe (131, 231) ist.

12. Maschine nach einem der Ansprüche 8 oder 9, wobei
die Maschine ein Industrieroboter (100) ist,
der Maschinen-Körper einen Arm (112) sowie ein Untersetzungsgetriebe (131, 231) einschließt, das an einem Gelenkabschnitt (130) des Arms (112) eingesetzt wird,
das Schmiermittel (131 a) ein Schmiermittel für das Untersetzungsgetriebe (131, 231) ist, der Arm (112) der Abschnitt an der Seite des Sensors ist,
das Untersetzungsgetriebe (131, 231) der Abschnitt an der Seite des Reinigungselementes ist, und
das Reinigungselement (280, 290) an einer Position angeordnet ist, an der es mit den den Spalt bildenden Flächen in Kontakt ist, wenn sich das Untersetzungsgetriebe (131, 231) dreht.

## Revendications

1. Capteur de détérioration de lubrifiant (137a, 137b, 139a, 139b, 237, 239) pour détecter la détérioration du lubrifiant (131a) d'un corps de machine dans un état où le capteur de détérioration de lubrifiant (137a, 137b, 139a, 139b, 237, 239) est monté dans le corps de machine, le capteur de détérioration de lubrifiant (137a, 137b, 139a, 139b, 237, 239) comprenant :

un élément d'émission de lumière (72) configuré pour émettre de la lumière ;
un élément de réception de lumière colorée (73) configuré pour détecter la couleur de la lumière reçue ;
un élément de formation d'intervalle (60) formant un intervalle d'huile (60a) dans lequel entre le lubrifiant (131a) ;
un élément de support (30) supportant l'élément d'émission de lumière (72), l'élément de réception de lumière colorée (73) et l'élément de formation d'intervalle (60) ; et
un élément de fixation (20) configuré pour être fixé au corps de machine,
dans lequel
l'élément de formation d'intervalle (60) est configuré pour transmettre la lumière émise par l'élément d'émission de lumière (72),
l'intervalle d'huile (60a) est disposé sur un chemin optique (10a) allant de l'élément d'émission de lumière (72) à l'élément de réception de lumière colorée (73),
l'élément de fixation (20) comprend une portion de contact (21) qui est configurée pour entrer en contact avec le corps de machine sur un côté externe de la portion de contact (21), et
le capteur de détérioration de lubrifiant étant **caractérisé en ce que**
l'élément de support (30) est disposé sur une côté interne de la portion de contact (21) de manière à être séparé de la portion de contact (21) par un intervalle (10b) tel qu'au moins une partie du chemin optique (10a) se trouve du côté interne de la portion de contact (21).

2. Capteur de détérioration de lubrifiant (137a, 137b, 139a, 139b, 237, 239) selon la revendication 1, dans lequel la portion de contact (21) est configurée pour être insérée dans un trou (113a) du corps de machine.

3. Capteur de détérioration de lubrifiant (137a, 137b, 139a, 139b, 237, 239) selon la revendication 2, dans lequel le trou (113a) est un trou de vis, et
la portion de contact (21) est une portion de vis qui est configurée pour être insérée dans le trou de vis (113a) du corps de machine et fixée à celui-ci.

4. Capteur de détérioration de lubrifiant (137a, 137b, 139a, 139b, 237, 239) selon l'une des revendications 1 à 3, dans lequel
l'élément de fixation (20) supporte l'élément de support (30) de manière rotative de telle sorte que la direction d'une ouverture (60b) de l'intervalle d'huile (60a) change quand l'élément de support (30) tourne.

5. Capteur de détérioration de lubrifiant (137a, 137b, 139a, 139b, 237, 239) selon la revendication 4, dans lequel l'élément de support (30) comprend une portion de réception de la force d'entraînement par rotation (51) qui reçoit une force d'entraînement par rotation par rapport à l'élément de fixation (20) provenant de l'extérieur via une force de contact, dans une position où la portion de réception de la force d'entraînement par rotation (51) n'entre pas en contact avec le lubrifiant (131a) dans un état où l'élément de fixation (20) est fixé au corps de machine.

6. Capteur de détérioration de lubrifiant (137a, 137b, 139a, 139b, 237, 239) selon la revendication 4 ou 5, comprenant en outre :

un élément d'empêchement de rotation (12) qui entre en contact aussi bien avec l'élément de support (30) qu'avec l'élément de fixation (20) de manière à éviter une rotation de l'élément de support (30) par rapport à l'élément de fixation (20), dans lequel
l'élément d'empêchement de rotation (12) comprend une portion de réception de la force d'entraînement par contact (12a) qui reçoit une force d'entraînement pour entrer en contact aussi bien avec l'élément de support (30) qu'avec l'élément de fixation (20) provenant de l'extérieur via une force de contact, dans une position où la portion de réception de la force d'entraînement par contact (12a) n'entre pas en contact avec le lubrifiant (131a) dans un état où l'élément de fixation (20) est fixé au corps de machine.

7. Machine comprenant :

le capteur de détérioration de lubrifiant (137a, 137b, 139a, 139b, 237, 239) selon l'une des revendications 1 à 6 ; et
le corps de machine.

8. Machine selon la revendication 7, comprenant en outre :

un élément de nettoyage (280, 290) configuré pour nettoyer le capteur de détérioration de lubrifiant (137a, 137b, 139a, 139b, 237, 239), dans laquelle
le corps de machine comprend une portion côté capteur (112, 234) sur laquelle est monté le capteur de détérioration de lubrifiant (137a, 137b, 139a, 139b, 237, 239) et une portion côté élément de nettoyage (234, 235b) qui est mobile par rapport à la portion côté capteur (112, 234) et sur laquelle est monté l'élément de nettoyage (280, 290), et dans laquelle
l'élément de nettoyage (280, 290) est disposé dans une position de contact avec des surfaces de formation d'intervalle de l'élément de formation d'intervalle (60) formant l'intervalle d'huile (60a) quand la portion côté capteur (112, 234) et la portion côté élément de nettoyage (234, 235b) se déplacent l'une par rapport à l'autre.

9. Machine selon la revendication 8, dans laquelle
l'élément de nettoyage (280, 290) comprend une pluralité de portions d'insertion (281) configurées pour être insérées dans l'intervalle d'huile (60a) et présentant une élasticité,
une largeur de chacune des portions d'insertion (281) en direction de la distance de l'intervalle d'huile (60a) est inférieure à la distance de l'intervalle d'huile (60a), et
une largeur entière de la pluralité de portions d'insertion (281) en direction de la distance de l'intervalle d'huile (60a) est supérieure à la distance de l'intervalle d'huile (60a).

10. Machine selon l'une des revendications 7 à 9, dans laquelle
la machine est un réducteur (131, 231) pour un robot industriel (100), et
le corps de machine est un corps principal du réducteur (131, 231).

11. Machine selon l'une des revendications 7 à 9, dans laquelle
la machine est un robot industriel (100),
le corps de machine comprend un bras (112) et un réducteur (131, 231) utilisés comme portion d'articulation (130) du bras (112), et
le lubrifiant (131a) est un lubrifiant pour le réducteur (131, 231).

12. Machine selon la revendication 8 ou 9, dans laquelle

la machine est un robot industriel (100),

le corps de machine comprend un bras (112) et un réducteur (131, 231) utilisés comme portion d'articulation (130) du bras (112),

le lubrifiant (131a) est un lubrifiant pour le réducteur (131, 231),

le bras (112) est la portion côté capteur,

le réducteur (131, 231) est la portion côté élément de nettoyage, et

l'élément de nettoyage (280, 290) est disposé dans une position de contact avec les surfaces de formation d'intervalle quand le réducteur (131, 231) est en rotation.

*Fig. 1*

Fig. 2

Fig. 3

*Fig. 4*

*Fig. 5*

( a )

( b )

*Fig. 6*

( a )

( b )

# Fig. 7

(a)

(b)

## Fig. 8

(a)

(b)

*Fig. 9*

( a )

( b )

*Fig. 10*

(a)

(b)

# Fig. 11

( a )

( b )

Fig. 12

## Fig. 13

( a )

( b )

*Fig. 14*

(a)

(b)

Fig. 15

# Fig. 16

( a )

( b )

( c )

## Fig. 17

LUBRICANT CONTAINING Mo-BASED ADDITIVE

| RELATED CONVERSION TIME | COLOR DIFFERENCE $\Delta E$ | |
|---|---|---|
| | SAMPLING MEASUREMENT | REAL TIME MEASUREMENT |
| 0 | 360.0 | 358.0 |
| 254 | 334.0 | 308.0 |
| 358 | 328.0 | 293.0 |
| 616 | 317.0 | 252.0 |
| 875 | 311.0 | 213.0 |

(a)

(b)

*Fig. 18*

LUBRICANT NON CONTAINING Mo-BASED ADDITIVE

| RELATED CONVERSION TIME | COLOR DIFFERENCE ΔE | |
|---|---|---|
| | SAMPLING MEASUREMENT | REAL TIME MEASUREMENT |
| 0 | 440.6 | 438.2 |
| 233 | 421.8 | 425.2 |
| 669 | 428.2 | 408.0 |
| 929 | 425.7 | 410.0 |

(a)

(b)

Fig. 19

Fig. 20

Fig. 21

280

281

(a)

280

281

(b)

*Fig. 22*

(a)

(b)

*Fig. 23*

EP 2 816 343 B1

*Fig. 24*

47

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7146233 A
- JP 10104160 A
- US 2008024761 A1
- US 2009216464 A1
- JP 2012032739 A

### Non-patent literature cited in the description

- *Faculty of Engineering, Research Report,* March 2003, vol. 51 (1), 81-88
- **TOMOMI HONDA.** DETERIORATION DIAGNOSIS OF LUBRICANT •INSPECTION TECHNOLOGY. *Journal of Japan Society for Precision Engineering,* 2009, vol. 75 (3), 359-362